# EUROPEAN PATENT SPECIFICATION

(11) **EP 2 138 545 B1**
(45) Date of publication and mention of the grant of the patent: **08.01.2014**
(21) Application number: 08721505.9
(22) Date of filing: 06.03.2008
(51) Int. Cl.: C09B 47/00, C07D 333/28, C07D 495/22, H01L 29/786, H01L 51/05, H01L 51/30, H01L 51/50

(54) **USE OF PORPHYRAZINE DERIVATIVE AS SEMICONDUCTOR**
VERWENDUNG EINES PORPHYRAZINDERIVATS ALS HALBLEITER
UTILISATION D'UN DERIVE DE PORPHYRAZINE COMME SEMICONDUCTEUR

(30) Priority: 07.03.2007 JP 2007057845
(43) Date of publication of application: 30.12.2009
(60) Divisional application: 13164291.0
(73) Proprietor: Hiroshima University, Hiroshima 739-8511 (JP); NIPPON KAYAKU KABUSHIKI KAISHA, Tokyo 102-8172 (JP)
(72) Inventor: TAKIMIYA, Kazuo, Higashi-Hiroshima-shi, 739-8527 Hiroshima (JP); MIYAZAKI, Eigo, Higashi-Hiroshima-shi, 739-8527 Hiroshima (JP)
(74) Representative: Bittner, Thomas L.
(86) International application number: PCT/JP2008/054085
(87) International publication number: WO 2008/108442

(56) References cited:
- JP-A- 2 035 462
- JP-A- 03 013 382
- JP-A- 61 291 187
- JP-A- 2000 072 975
- JP-A- 2006 143 680
- ISHII A. ET AL.: 'Syntheses and reactions of 4- and 8-phosphatiophenetriptycenes' TETRAHEDRON LETTERS vol. 34, no. 51, 1993, pages 8255 - 8258, XP008118018
- KUDOH K. ET AL.: 'Reactions of fused polycyclic 1,2-dithiins with transition metals: synthesis of heteroacenes via desulfurization' ORGANOMETALLICS vol. 25, no. 9, 2006, pages 2374 - 2377, XP008118020
- GRONOWITZ S. ET AL.: 'Ring-opening reactions. XVII. On the reaction of 4-chloro-5-methoxythiophene and its monobromo derivatives with butyllithium' CHEMICA SCRIPTA vol. 15, no. 1, 1980, pages 1 - 3, XP008118102
- SUGIE A. ET AL.: 'Observation of sequential electrophilic substitution of bromothiophene and immediate reductive elimination of arylpalladium complexes' CHEMISTRY LETTERS vol. 35, no. 10, 2006, pages 1100 - 1101, XP008118103

## Description

### Technical Field

The present invention relates to uses of porphyrazine derivatives. The porphyrazine derivative has a high solubility in an organic solvent and is excellent in molecular orientation. Also disclosed is a method for production of the porphyrazine derivative and the intermediate, and uses of derivative and the intermediate.

### Background Art

Phthalocyanine, in which porphyrazine is condensed with four phenyl groups, has a unique electron system and is widely used as a functional material including a blue pigment for a display color filter, an organic photoconductor of a copying machine, and the like. In this way, phthalocyanine is known to have a high degree of potential as a functional molecule. In order to more efficiently realize such potential of phthalocyanine, there has been made an attempt to combine a phthalocyanine skeleton with a thiophene skeleton so that a carrier transport capacity is improved (Patent Literature 1, Non Patent Literatures 1 and 2).

Most phthalocyanine derivatives have a high melting point and are highly stable to heat. However, the phthalocyanine derivatives are poorly soluble in most organic solvents due to its strong intermolecular interaction, and therefore are difficult to use in a solution process. Because of such a problem, the phthalocyanine derivatives are used generally with a method for suspending a phthalocyanine derivative with a polymer, a method for vacuum depositing a phthalocyanine derivative at a high temperature, or other method. For the purpose of improving solubility in an organic solvent and applying as a liquid crystal material, there also has developed phthalocyanine into which a number of alkoxy groups are introduced (Non Patent Literature 2).

Further, Patent Literature 2 discloses optical recording media that include, as a light-absorbing substance, a compound having a structure in which thiophene having a methyl group is condensed with porphyrazine and a metal complex is formed.

### Citation List

Patent Literature 1
   Japanese Patent Application Publication, Tokukai, No. 2006-143680
Patent Literature 2
   Japanese Patent Application Publication, Tokukaisho, No. 61-291187
Non Patent Literature 1
   Douglas M. Knawby and Timothy M. Swager, Chem. Mater. 1997, 9, p535-538
Non Patent Literature 2
   Michael J. Cook and Ali Jafari-Fini, Tetrahedron 56 (2000) P4085-4094

JP 2006-143680 discloses compounds having a phthalocyanine moiety conjugated with an oligothiophene moiety.

JP 61-29/187 discloses phthalocyanine derivatives comprising condensed thiophenes moieties.

### Summary of Invention

However, introduction of a long-chain alkoxy group strengthens a van der Waals force between alkyl groups. This makes it difficult for molecules to be regularly arrayed. As a result, it becomes difficult to form a crystalline thin film. This causes a disadvantage in expression of an electronic property that requires a high molecular orientation (a high crystallinity). This also becomes an obstacle to produce a high-performance functional device. Therefore, there has been a demand for a phthalocyanine derivative (or porphyrazine derivative) having a high solubility in an organic solvent and being capable of forming a crystalline thin film.

The present invention has been accomplished in view of the problem, and an object of the present invention is to provide a novel porphyrazine derivative and an intermediate thereof, which porphyrazine derivative has a high solubility in an organic solvent and a high molecular orientation, a method for production of the porphyrazine derivative and the intermediate, and use of the porphyrazine derivative and the intermediate.

The inventor of the present invention has been diligently studied in order to attain the object. As a result, it was found that a porphyrazine derivative in which an alkyl group or the like is introduced into a certain position of a thiophene skeleton that forms a condensed ring with porphyrazine has a high solubility in an organic solvent and is excellent in molecular orientation. Such a derivative was found to satisfy, at a high level, both of simplicity of use in a solution process and properties of a device such as an organic thin-film transistor. Based on these findings, the inventor accomplished the present invention.

The present invention refers to the use of a porphyrazine derivative as an organic semiconductor material in accordance with the appended claims.

A compound according to the present invention is a compound being a porphyrazine derivative forming a condensed ring with thiophene having a skeleton which has, at position 2, an alkyl group, an alkoxy group, the alkyl group being a C₁-C₃₀ alkyl group whose one or more carbon atoms may be substituted with an oxygen atom, an nitrogen atom, and/or a sulfur atom.

As a porphyrazine derivative having a thiophene skeleton, Patent Literature 1, for example, refers to a compound having at least a phthalocyanine skeleton and an oligothiophene skeleton, the phthalocyanine skeleton being conjugated with the oligothiophene skeleton. This compound, in which π electron system of the phthalocyanine skeleton is conjugated with that of the thiophene skeleton, has a high carrier transport capacity and a high light absorbing capacity.

However, this compound is insufficiently soluble in an organic solvent and therefore has difficulty in forming a film with a solution process. It is considered that such a problem is attributed to a strong intermolecular interaction that is caused because there exists no soluble substitution group and conjugated π electrons widely distribute in a same plane.

The compound in accordance with the present invention has a smaller amount of thiophene in a molecule than the compound of Patent Literature 1, and has an alkyl group or the like introduced into a certain position (position 2 of a thiophene skeleton). Therefore, the compound in accordance with the present invention has a structure that molecules are regularly arrayed without any steric hindrance caused by the alkyl group or the like in a molecule. This allows the compound in accordance with the present invention to have a high solubility in an organic solvent and to exhibit a high crystallinity. Therefore, the compound in accordance with the present invention can be suitably used in formation of a crystalline thin film with a solution process.

Further, in the compound in accordance with the present invention, more than one phenyl groups in a phthalocyanine molecule are substituted by thiophene. Therefore, like the compound of Patent Literature 1, the compound in accordance with the present invention has a high carrier transport capacity and a high light absorbing capacity, each of which is attributed to the phthalocyanine skeleton, i.e., porphyrazine, and thiophene. This makes it possible for the compound in accordance with the present invention to have a high potential as an organic semiconductor material and a high solubility in an organic solvent. Therefore, it is possible to provide a material which is useful in practical application and the like of various organic FET elements with use of a solution process.

Moreover, a compound according to the present invention is a compound represented by the following general formula (1): 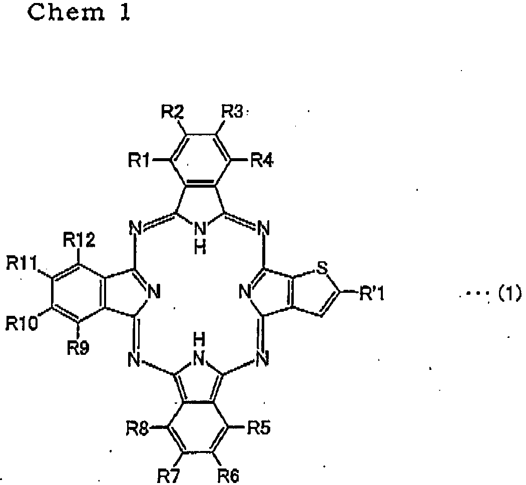 where:
R1 to R12 are independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
R'1 is an alkyl group, the alkyl group being a C₁-C₃₀ alkyl group whose one or more carbon atoms may be substituted with an oxygen atom, an nitrogen atom, and/or a sulfur atom.

According to this arrangement, one benzene ring in a phthalocyanine molecule is substituted by thiophene, and an alkyl group is introduced into a certain position of the thiophene. This makes it possible to attain a high carrier transport capacity and a high light absorbing capacity, each of which is attributed to porphyrazine and thiophene, and a high solubility in an organic solvent. Therefore, it is possible to provide a material which is useful in practical application of various organic FET elements with use of a solution process.

Furthermore, a compound according to the present invention is a compound represented by the following general formula (2): 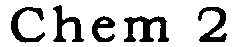 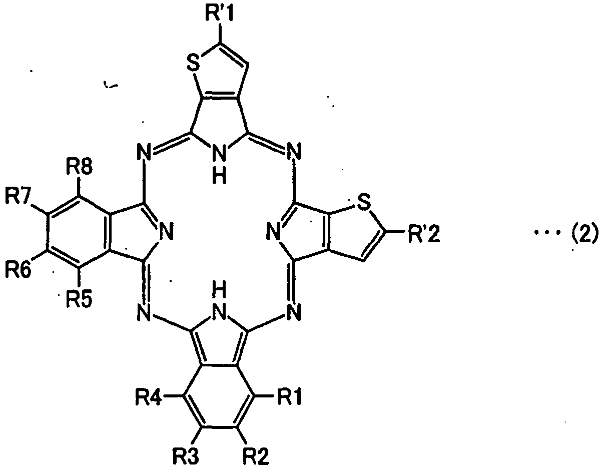 where:
R1 to R8 are independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
R'1 and R'2 are independently an alkyl group, the alkyl group being a C₁-C₃₀ alkyl group whose one or more carbon atoms may be substituted with an oxygen atom, an nitrogen atom, and/or a sulfur atom.

According to this arrangement, two benzene rings in a phthalocyanine molecule are substituted by thiophene, and an alkyl group or the like is introduced into a certain position of the thiophene. This makes it possible to attain a high carrier transport capacity and a high light absorbing capacity, each of which is attributed to porphyrazine and thiophene, and a high solubility in an organic solvent. Therefore, it is possible to provide a material which is useful in practical application of various organic FET elements with use of a solution process.

Moreover, a compound according to the present invention is a compound represented by the following general formula (3): 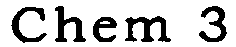 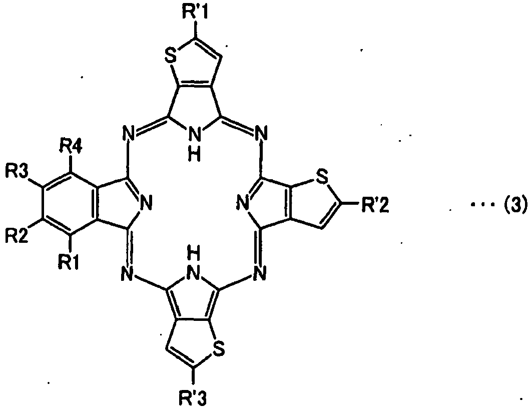 where R1 to R4 are independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
R'1 to R'3 are independently an alkyl group, the alkyl group being a C₁-C₃₀ alkyl group whose one or more carbon atoms may be substituted with an oxygen atom, an nitrogen atom, and/or a sulfur atom.

According to this arrangement, three benzene rings in a phthalocyanine molecule are substituted by thiophene, and an alkyl group or the like is introduced into a certain position of the thiophene. This makes it possible to attain a high carrier transport capacity and a high light absorbing capacity, each of which is attributed to a porphyrazine skeleton and thiophene, and a high solubility in an organic solvent. Therefore, it is possible to provide a material which is useful in practical application of various organic FET elements with use of a solution process.

Furthermore, a compound according to the present invention is a compound represented by the following general formula (4): 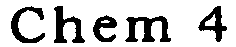 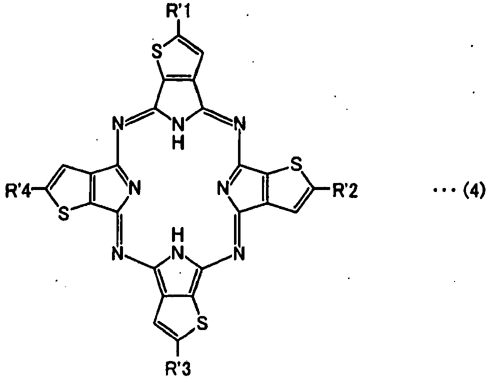 where R'1 to R'4 are independently an alkyl group, the alkyl group being a C₁-C₃₀ alkyl group whose one or more carbon atoms may be substituted with an oxygen atom, an nitrogen atom, and/or a sulfur atom.

Moreover, a compound according to the present invention is a compound represented by any one of the following chemical formula (5), (6), and (7): 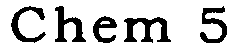 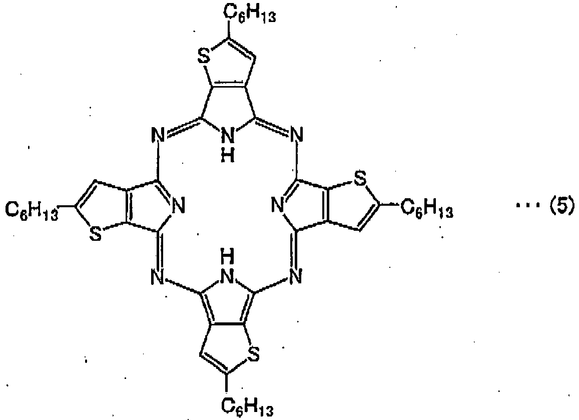 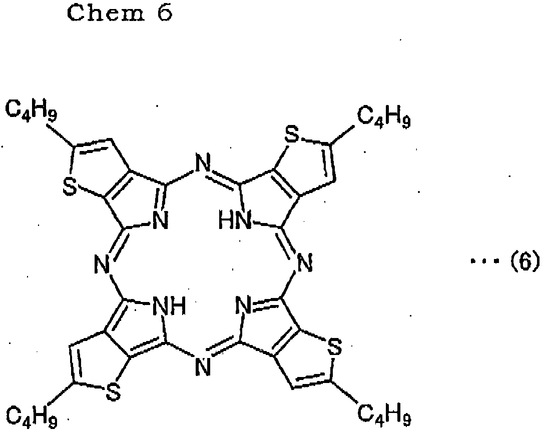 and

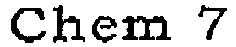

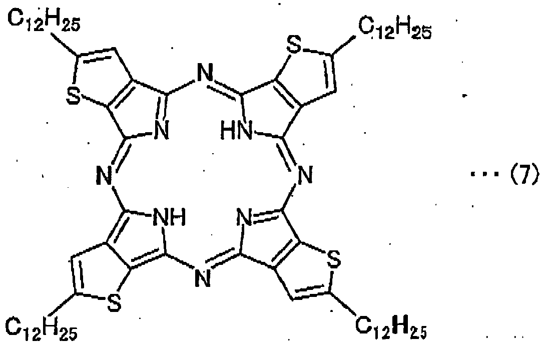

According to this arrangement, four benzene rings in a phthalocyanine molecule are substituted by thiophene, and a hexyl group, a butyl group, or a dodecyl group is introduced into a certain position of the thiophene. This makes it possible to attain a high carrier transport capacity and a high light absorbing capacity, each of which is attributed to porphyrazine and thiophene, and a high solubility in an organic solvent. Therefore, it is possible to provide a material which is useful in practical application of various organic FET elements with use of a solution process.

Furthermore, a compound according to the present invention is any one of the compounds according to the present invention, wherein the porphyrazine is a metal complex.

Porphyrazine has a characteristic of forming a stable complex with a metal ion. Formation of such a metal complex allows porphyrazine to have an electric conduction property, magnetism, and a semiconductor property. It should be noted that "porphyrazine" herein includes porphyrazine itself and a moiety having a porphyrazine structure in a compound, such as compounds represented by the general formulas 1 through 4, in which a functional group such as a phenyl group and thiophene is condensed with porphyrazine. The word "porphyrazine" herein is used synonymously with "phthalocyanine skeleton".

Also disclosed is a compound represented by the following general formula (8): 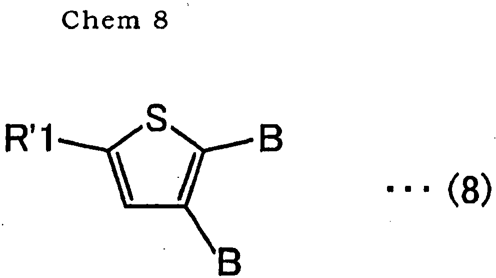 where B is a halogen atom, and R'1 is an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom.

Moreover, the compound is arranged such that B is bromine, and R'1 is a hexyl group, a butyl group, or a dodecyl group.

It is possible to produce compounds represented by the general formulas (1) through (4) from this compound by substituting halogen with a cyano group and closing a ring in the presence of lithium. Therefore, it is possible to use this compound as an intermediate of the compound in accordance with the present invention.

Also disclosed is a method of producing a compound represented by the following general formula (8): 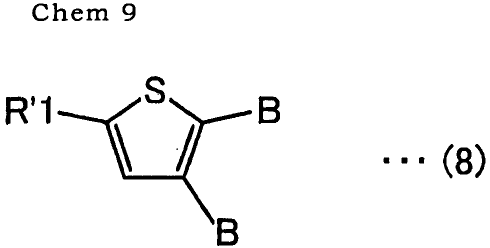 the method comprising:
a first step for reacting a compound represented by the following general formula (9) with an organic metal compound or a base: 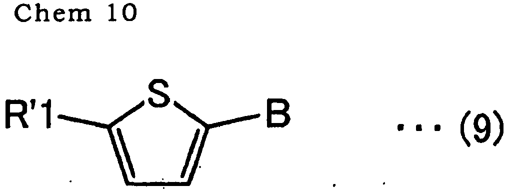
and then
a second step for reacting a product of the first step with a protonic solvent,
where:
in chemical formulae (8) and (9), R'1 is an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
B is a halogen atom.

Also disclosed is a method as set forth above, wherein in chemical formulae (8) and (9), B is bromine, and R'1 is a hexyl group, a butyl group, or a dodecyl group.

As described later, it is possible to produce compounds represented by the general formulas (1) through (4) by substituting halogen of a compound represented by the general formula (8) with a cyano group and further by closing a ring in the presence of lithium. The compound represented by the general formula (8) can be obtained by halogenation at position 3 of a compound represented by the general formula (9). However, it is extremely difficult to directly cause the halogenation at position 3. In contrast, it is comparatively easy to cause halogenation at position 2 of the compound represented by the general formula (9).

In the method the compound represented by the general formula (9) is reacted with an organic metal compound or a base. This makes it possible to transfer halogen from position 2 to position 3 of the compound represented by the general formula (9). By causing halogenation at position 2 after the transfer, it is possible to easily produce the compound represented by the general formula (8). Also disclosed is a method for production of a compound in accordance with the present invention, characterized by including a method for production of a compound represented by the general formula (8).

With this arrangement, it is possible to easily produce the compound represented by the general formula (8), which compound is an intermediate of the compound in accordance with the present invention. Accordingly, it is also possible to easily produce the compound in accordance with the present invention. Therefore, it is possible to easily provide at low cost a material which is useful in practical application of various organic FET elements with use of a solution process. The device in accordance with the present invention includes a compound in accordance with the present invention, which the device according to the present invention is a photoelectric conversion element, a solar battery, a dye-sensitized solar cell element, an organic EL element, a liquid crystal display element, an organic thin-film transistor element, or a light-emitting device having an organic carrier transport layer.

The device is preferably arranged such that the organic thin-film transistor element is a top-contact type element. The device is preferably arranged such that the organic thin-film transistor element is a bottom-contact type element.

The compound in accordance with the present invention can be suitably subjected to a solution process because of being soluble in an organic solvent as described above, and has a high molecular orientation. Therefore, the compound in accordance with the present invention is highly advantageous in expression of an electronic property. This makes it possible to produce the device at low cost with simple processes. Further, the device can have a large area and has an extremely high potential.

Additional objects, features, and strengths of the present invention will be made clear by the description below. Further, the advantages of the present invention will be evident from the following explanation in reference to the drawings.

### Brief Description of Drawings

Fig. 1

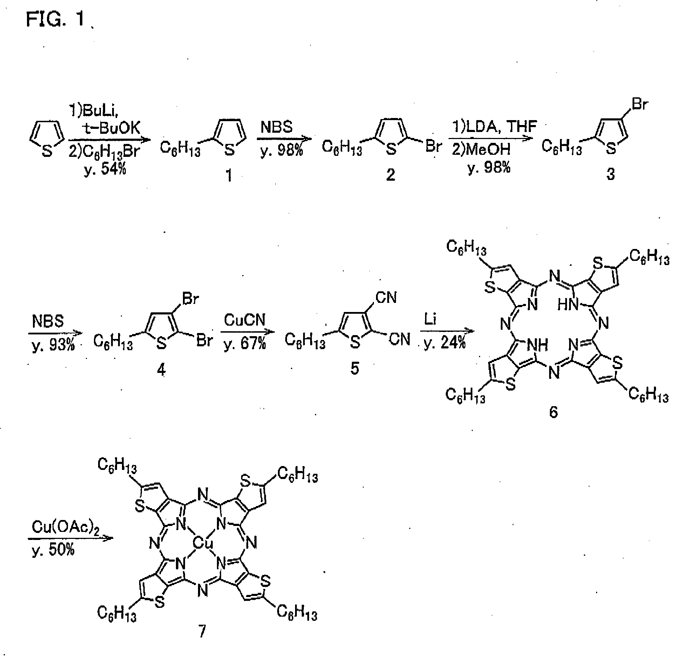

   Fig. 1 is a view illustrating an example of production scheme of a compound shown in Examples in accordance with the present invention.
Fig. 2

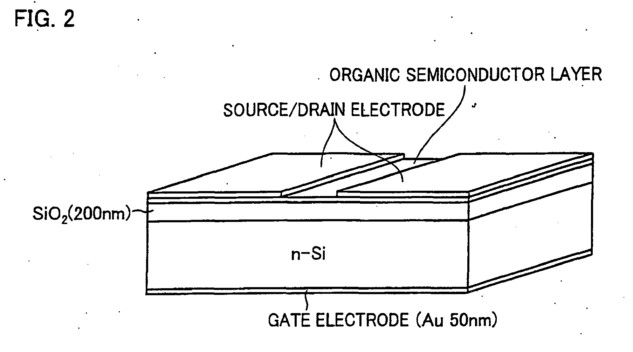

   Fig. 2 is a schematic view illustrating a structure of an FET element produced with a compound indicated by "6" in Fig. 1.
Fig. 3

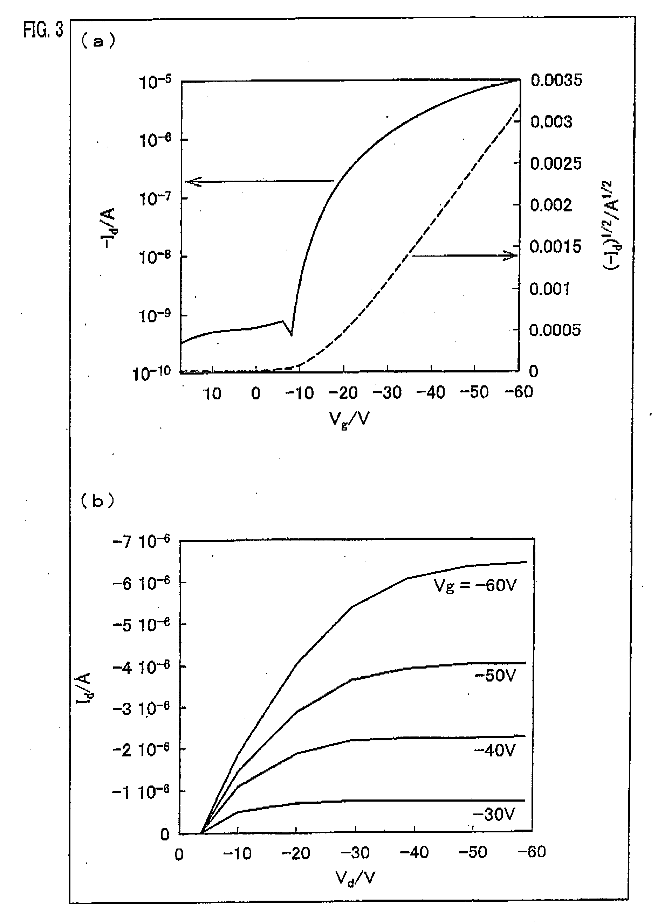

   Fig. 3 is a graph showing FET characteristics of an element (i) produced with a silicon substrate having been treated with OTS and (ii) subjected to annealing at 100°C. In Fig. 3, (a) and (b) show an Id-Vg characteristic and an Id-Vd characteristic, respectively.
Fig. 4

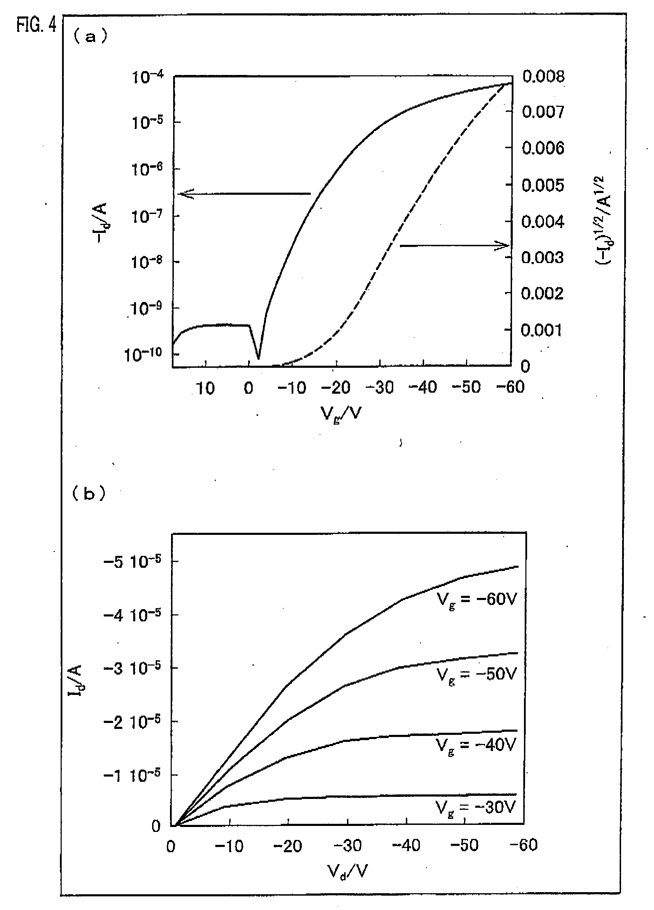

   Fig. 4 is a graph showing FET characteristics of an element (i) produced with a silicon substrate having been treated with HMDS and (ii) subjected to annealing at 120°C. In Fig. 4, (a) and (b) show an Id-Vg characteristic and an Id-Vd characteristic, respectively.
Fig. 5

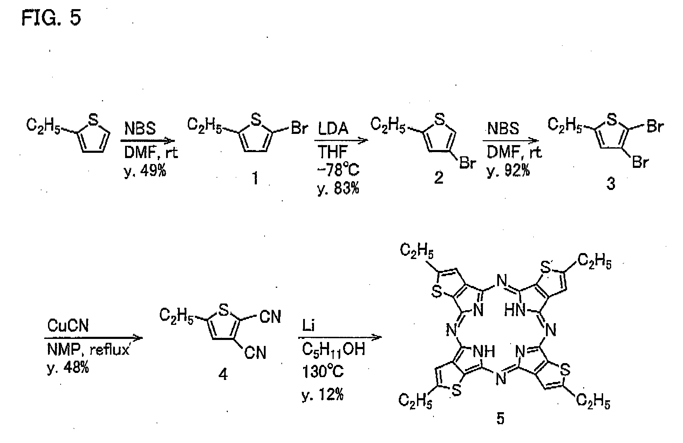

   Fig. 5 is a view illustrating a production scheme of another example of a compound in accordance with the present invention.
Fig. 6

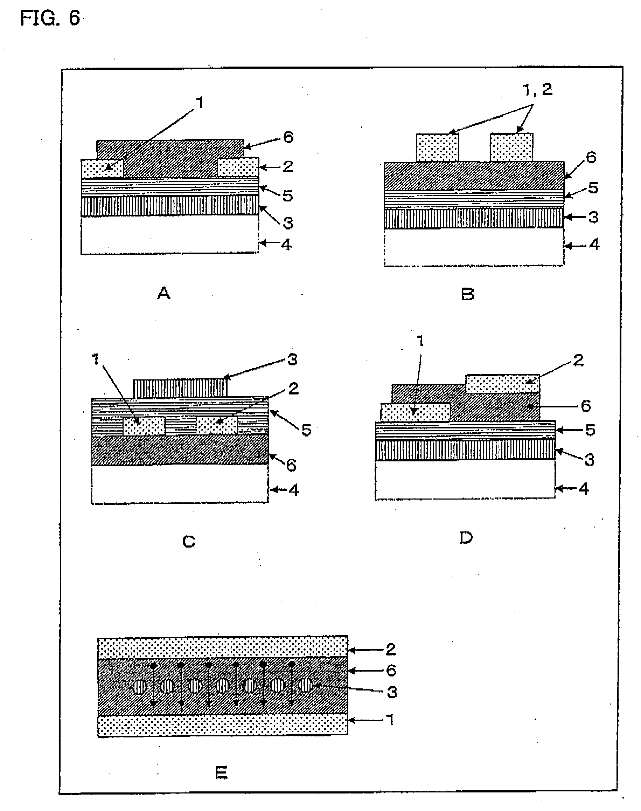

   Fig. 6 is a schematic view illustrating some examples of an organic thin-film transistor element that includes a compound in accordance with the present invention.
Fig. 7

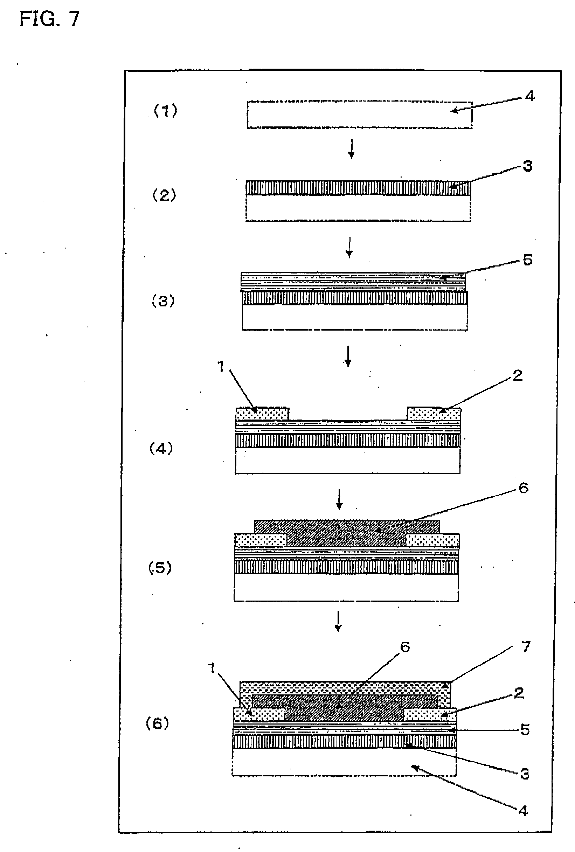

   Fig. 7 is a schematic view illustrating a production method of a bottom-contact type organic thin-film transistor element indicated by A in Fig. 6.
Fig. 8

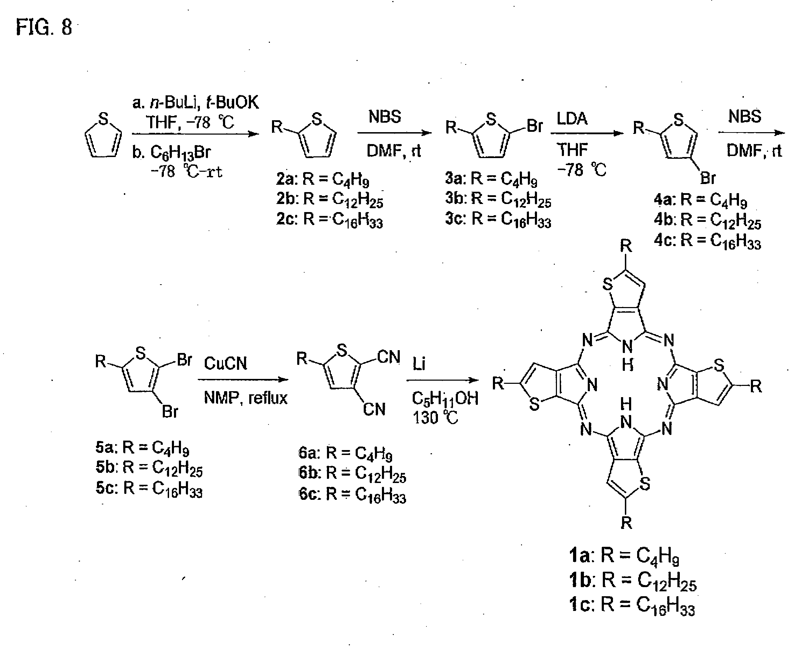

   Fig. 8 is a view illustrating a production scheme of a porphyrazine derivative, which is another example of a compound in accordance with the present invention, having a different chain length of an alkyl group from the other example of the compound in accordance with the present invention.
Fig. 9

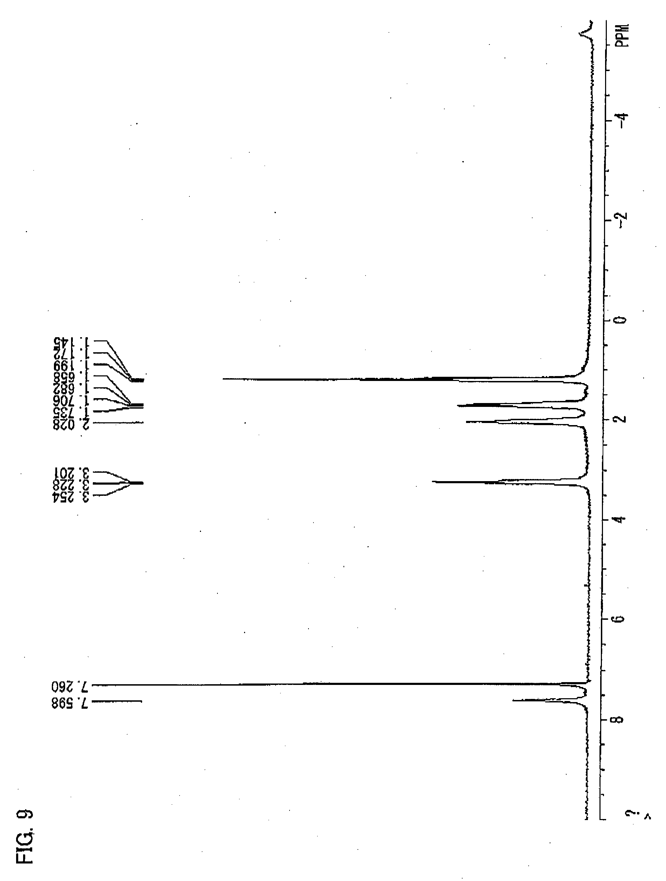

   Fig. 9 is a view showing an NMR spectrum of a chloroform-d solution of a porphyrazine derivative (Tetrakis(5-butylthiopheno)[2.3-b]porphyrazine) indicated by 1a.
Fig. 10

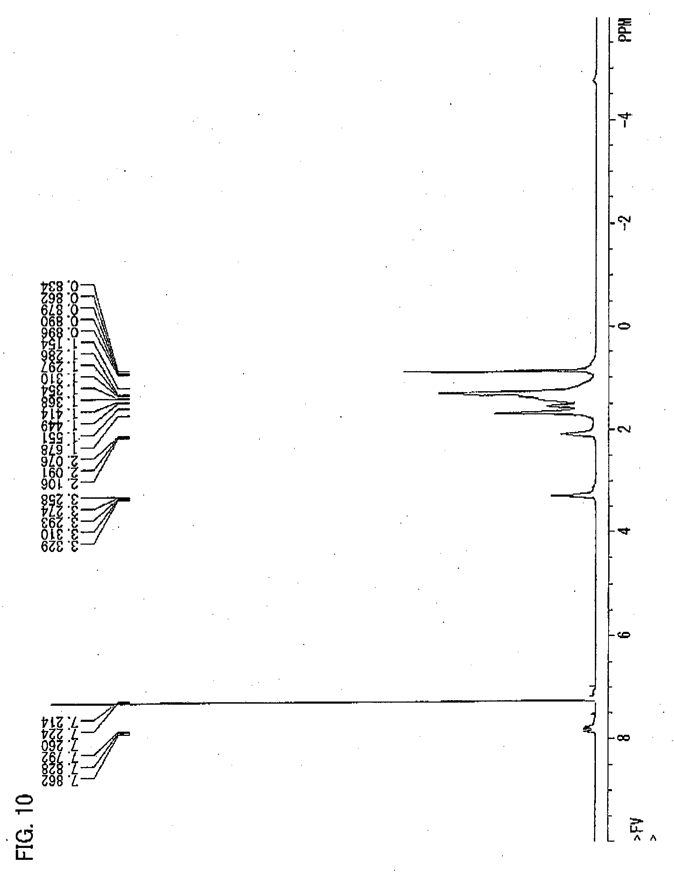

   Fig. 10 is a view showing an NMR spectrum of a chloroform-d solution of a porphyrazine derivative (Tetrakis(5-dodecylthiopheno)[2.3-b]porphyrazine) indicated by 1b.
Fig. 11

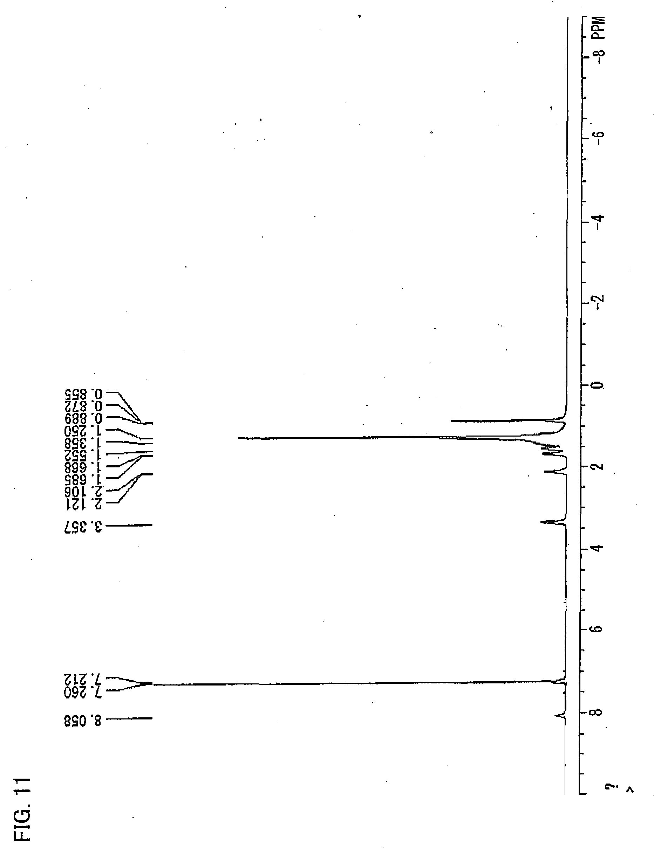

   Fig. 11 is a view showing an NMR spectrum of a chloroform-d solution of a porphyrazine derivative (Tetrakis(5-cetylthiopheno)[2.3-b]porphyrazine) indicated by 1c.

### Reference Signs List

1: SOURCE ELECTRODE
2: DRAIN ELECTRODE
3: GATE ELECTRODE
4: SUBSTRATE
5: DIELECTRIC LAYER
6: ORGANIC SEMICONDUCTOR MATERIAL
7: PROTECTION LAYER

### Description of Embodiments

The present invention relates to uses of novel porphyrazine derivatives in accordance with the appended claims. Therefore, the following descriptions refer to firstly a compound in accordance with the present invention, secondly a method for production of the compound and an intermediate thereof, and lastly use of the compound and the intermediate.

### <1. Compound in accordance with the present invention>

A compound according to the present invention is a compound being a porphyrazine derivative forming a condensed ring with thiophene having a skeleton which has, at position 2, an alkyl group, the alkyl group being a C₁-C₃₀ alkyl group whose one or more carbon atoms may be substituted with an oxygen atom, an nitrogen atom, and/or a sulfur atom.

The porphyrazine has to form a condensed ring with thiophene. Porphyrazine can form a condensed ring with thiophene at respective four positions between position 2 and position 3, between position 7 and position 8, between position 12 and position 13, and between position 17 and position 18. However, the number of thiophene to be condensed and which position to be condensed are not particularly limited, provided that one porphyrazine is condensed with at least one thiophene. As described in Patent Literature 1, increase in number of oligothiophene skeletons to be conjugated with porphyrazine (phthalocyanine skeleton) causes improvement of carrier transport capacity and light absorbing capacity. In view of this, the number of thiophene to be condensed with one porphyrazine is preferably two rather than one, more preferably three rather than two, further preferably four rather than three.

In a case where one porphyrazine further includes a condensed ring other than thiophene, a type of the condensed ring is not particularly limited. For example, in a case where one thiophene is condensed with one porphyrazine at a position between position 2 and position 3, a type of a condensed ring formed, at a different position, other than thiophene is not particularly limited. The condensed ring other than thiophene may be, for example, a benzene ring, naphthalene, anthracene, pyridine, pyrazine, pyrrole, furan, or thiophene,S,S-dioxide.

At position 2, a skeleton of the thiophene includes a functional group such as an alkyl group. The "skeleton of the thiophene" herein is a structure represented by the general formula (10). The "position 2" means a position next to where a sulfur atom is in the general formula (10).

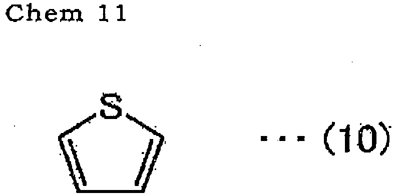

Binding a functional group such as an alkyl group to position 2 of the skeleton of the thiophene makes it possible to prevent a contact between functional groups, thereby preventing a steric hindrance. This allows the functional group such as the alkyl group to sufficiently produce an effect of solubility improvement. Therefore, it is possible to significantly improve the compound in solubility in an organic solvent compared to, for example, the compound described in Patent Literature 1.

Further, it is possible to maintain at a high level a molecular orientation of the compound because the steric hindrance does not occur. This allows a high advantage in expression of an electronic property that requires a high crystallinity. Therefore, it is possible to sufficiently take advantage of stability and heat resistance of porphyrazine and a high electronic property obtained due to presence of porphyrazine and thiophene.

This finding is obtained by the present invention for the first time. For example, in a case where a functional group is bound to a position other than position 2, there occurs a contact between functional groups in the compound, thereby resulting in lowering of molecular orientation. In this case, even if the functional group can produce the effect of improving solubility in an organic solvent, the high electronic property cannot be obtained. That is to say, it is impossible to satisfy both of a high solubility in an organic solvent, which is essential for production of a device with a solution process, and a high functionality of a device.

The "solution process" is a method for disposing a semiconductor layer by coating a substrate with a solution that includes an organic semiconductor material. The "solution process" may be, for example, coating, spin coating, an ink-jet method, and the like. With such a method, it becomes possible to simplify production processes, significantly reduce costs of an apparatus and the like required for production, and produce a large-area organic semiconductor device. On these points, the solution process is highly useful. The compound in accordance with the present invention is excellent in electronic property and solubility in an organic solvent, and therefore can be an extremely suitable material for the solution process.

The compound may be, for example, a compound represented by any of the general formulas (1) through (4), more specifically, a compound represented by the chemical formula (5), (6), or (7). The compound represented by the chemical formula (5), (6) or (7) has an extremely high potential as a functional material. As described later in Examples, it was found that the compound represented by the chemical formula (5) exhibited a carrier mobility (p-type) of over 10⁻¹cm⁻²/Vs in the atmosphere when used in an organic FET element produced by spin coating. Therefore, the compound represented by the chemical formula (5) is considered to be particularly useful in practical application of various FET elements with use of a solution process.

In the general formulas (1) through (4), each of R1 through R12 may be independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom.

Each of R'1 through R'4 may be independently an alkyl group, the alkyl group being a C₁-C₃₀ alkyl group whose one or more carbon atoms may be substituted with an oxygen atom, an nitrogen atom, and/or a sulfur atom.

The alkyl group may be straight chains or may be branched. Among these functional groups, linear alkyl group is more preferable, from the point of view that, with the alkyl group, a steric hindrance between functional groups is less likely to occur and solubility in an organic solvent can be improved.

Each of R'1 through R'4 is the alkyl group among the functional groups above, more preferably an unsubstituted alkyl group. The alkyl group may be a straight chain, a branched chain, or in the form of a circle, preferably a straight chain or a branched chain, more preferably a straight chain. The number of carbons of the alkyl group is normally 1 to 30, preferably 4 to 30, more preferably 4 to 24, further preferably 6 to 20, particular preferably 6 to 16. It is preferable that each of R'1 through R'4 is independently the alkyl group. It is more preferable that R'1 through R'4 are same alkyl groups.

Porphyrazine has a characteristic of forming a stable complex with a metal ion. It will be understood that the present invention includes such a compound that porphyrazine of the compound in accordance with the present invention forms a complex with a metal ion. As the metal ion, a known metal ion used in formation of a phthalocyanine complex, such as iron, copper, nickel, and cobalt, can be suitably used. Formation of a complex with such a metal ion causes the compound to have properties such as an electric conduction property, magnetism, and a semiconductor property.

As described above, the compound in accordance with the present invention has both a high potential as an organic semiconductor material and a high solubility in an organic solvent. It was difficult to form a film by a solution process with a conventional compound because the conventional compound lacks in solubility in an organic solvent even if having a high potential. The compound in accordance with the present invention can be used as a material which has a high potential and can be subjected to the solution process. Therefore, the compound in accordance with the present invention is highly useful as a photoelectronic device material of, for example, a photoelectric conversion element, a solar battery, a dye-sensitized solar cell element, an organic EL element, a liquid crystal display element, an organic thin-film transistor element, or a light-emitting device having an organic carrier transport layer. In particular, because of ability to be subjected to the solution process, the compound can be applied to an electroinjection layer of an EL element, which has been extremely difficult to produce by the solution process, and an active layer of a solar battery.

### <2. Intermediate and production method

The compound described in the section <1> is a totally novel compound and synthesis thereof has not been reported. The compound could not have been synthesized because it was impossible to obtain a compound represented by the general formula (8). In other words, it can be said that the present invention was accomplished because the compound represented by the general formula (8) became obtainable.

It can be said that the compound represented by the general formula (8) is a useful intermediate of the compound in accordance with the present invention, which is described in the section <1>. Therefore, there is also disclosed this intermediate compound.

In one of methods for obtaining the compound in accordance with the present invention, which compound is described in the section <1>, the compound in accordance with the present invention is produced via the compound represented by the general formula (8). Therefore, a production method of the compound represented by the general formula (8) corresponds to one process of a production method of the compound in accordance with the present invention, and is highly useful. Also disclosed is the production method of this intermediate as well as the production method of the compound described in the section <1> which includes the production method of the intermediate.

The production method of the compound represented by the general formula (8) only has to include a first step for reacting a compound represented by the general formula (9) with an organic metal compound or a base, and a second step for reacting the resulting compound with a protonic solvent after the first step. Other steps, reaction conditions, a material, a catalyst, a production device, a production apparatus, and the like can be arranged as conventionally done, and are not particularly limited.

The compound represented by the general formula (9) has been known and can be easily obtained by a person skilled in the art with use of, for example, a method described in a literature (G. Barbarella et al., J. Org. Chem. 1998, 63, 5497).

In the step for reacting the compound represented by the general formula (9) with an organic metal compound or a base so that a halogen atom at position 2 of the compound represented by the general formula (9) is transferred, dehalogenation at position 2, at which halogenation is comparatively easy to occur, is firstly carried out so that the halogen is transferred from position 2 to position 3, and then halogenation at position 2 is carried out. If halogenation is carried out directly at position 3, a compound halogenated at position 4 is simultaneously or preferentially obtained. This causes a problem that the compound represented by the general formula (8) cannot be obtained with a high purity.

As the organic metal compound or the base, it is possible to suitably use, for example, LiTMP (lithium tetramethyltetramethylpyridine), LiHMDS (lithium hexamethyldisilazane), sodium hydride, potassium hydride, potassium-t-butoxide, sodium amide, potassium amide, and the like other than LDA (lithium diisopropylamide) which is used in Examples described later. However, the organic metal compound or the base is not limited to these reagents.

The organic metal compound or the base is used for the purpose of taking out a proton from position 2 of the compound represented by the general formula (9). The first step is not particularly limited in reaction conditions. For example, the compound represented by the general formula (9) reacts with the organic metal compound or the base when a mixture of them is stirred while being maintained at a temperature within a range of -100°C to 20°C. By this, the proton can be taken out from position 2 of the compound represented by the general formula (9). When used, the organic metal compound or the base can be dissolved in a known solvent such as THF, dimethylether, dimethoxyethane, dioxane, pentane, and hexane.

In the second step, a transfer reaction is stopped by addition of the protonic solvent.

The second step is not particularly limited in conditions. For example, the second step can be carried out by stirring a reacting system after the first step while maintaining a temperature of the reacting system within a range of -100°C to 20°C. The second step, which is intended to stop the reaction, can be carried out at a temperature within a range from a temperature at the end of the reaction to a room temperature. The protonic solvent is not particularly limited and may be a known lower or higher alcohol, water, or the like.

The compound represented by the general formula (8) can be obtained by reacting, by means of a known method, a halogenation reagent with a compound which is produced as above by transfer of a halogen atom from position 2 to position 3 of the compound represented by the general formula (9). The halogenation reagent may be a known halogenation reagent and a specific composition of the halogenation reagent is not particularly limited. For example, in a case where bromine is used as halogen, it is possible to use Br₂ or the like as well as N-bromosuccinimide (NBS) described later.

The halogen of the compound represented by the general formula (8) is not particularly limited, but is preferably bromine. The reason why bromine is preferable is that bromine compounds are more chemically stable than iodine compounds and therefore are easy to deal with and store. In this regard, chloride compounds are also more chemically stable than iodine compounds, however, are generally less active than bromine compounds in the subsequent cyanidation. Therefore, among compounds represented by the general formula (8), a compound in which the halogen is bromine is particularly suitable as an intermediate in the reaction of the present invention.

With the first and second steps, it is possible to efficiently obtain the compound represented by the general formula (8). It is considered that this time's success in obtaining the compound is achieved for the following reason. It is possible to synthesize the compound represented by the general formula (8) by introducing an alkyl group into a known compound having such a structure that an alkyl group is removed from the compound represented by the general formula (8). However, introduction of an alkyl group causes occurrence of not only the compound represented by the general formula (8) but also a mixture of the compound from which bromine is removed, the compound in which the alkyl group is introduced into where bromine exists, and the like. It is difficult to separate and purify these compounds.

In view of this, studied was a method for transferring a halogen atom from position 2 to position 3 of the compound represented by the general formula (9), and suitable reaction conditions of this method were determined. In Examples described later, a reaction was carried out with 2 equal amounts of approximately 0.5M LDA solution. However, in a case where an LDA solution of a lower concentration (approximately 0.3M) was used, 2 equal amounts of the LDA solution could not cause the reaction to proceed, and at least 5 equal amounts of the LDA solution was necessary to proceed the reaction. In this method, not only an equal amount of a reaction reagent but also a concentration of the reaction reagent was important.

Once the compound represented by the general formula (8) is obtained, it is possible to produce the compound described in the section <1> by a known organic chemical synthesis method. Specifically, based on a known literature (D. W. MacDowell et al., J. Org. Chem. 1977, 42, 3717) and the like, as described later in Examples, ring-closing is carried out in the presence of Li (lithium) with use of a number of dicyano compounds each obtained by substituting, with a cyano group, a halogen atom of the compound represented by the general formula (8). As a result, it is possible to obtain a compound having a desired structure, that is, a porphyrazine derivative in which porphyrazine forms a condensed ring with thiophene and a skeleton of the thiophene has a functional group such as an alkyl group at position 2.

Further, compounds represented by the general formulas (1) through (4) can be produced by, for example, (i) mixing at a certain ratio a phthalonitrile derivative with a dicyano compound obtained by substituting, with a cyano group, a halogen atom of the compound represented by the general formula (8), and then (ii) ring-closing in the presence of Li (lithium). According to a mixture ratio between the dicyano compound and the phthalonitrile derivative, it is possible to determine which one of the compounds represented by the general formulas (1) through (4) is preferentially produced among a number of products.

Japanese Patent Application Publication, Tokukai, No. 2000-72975 (publication date: March 7, 2000) discloses an electric charge production material formed from a porphyrazine derivative, and an electrophotographic photoreceptor. This application teaches that an alkyl group can be used as a substitution group.

However, the application does not describe at all such a feature of the compound in accordance with the present invention that the compound is a porphyrazine derivative in which porphyrazine forms a condensed ring with thiophene and a skeleton of the thiophene has a functional group such as an alkyl group at position 2. The invention of the application intends to provide an electric charge production material that is highly sensitive to irradiated light and allows a high dispersion stability of coating liquid, and an electrophotographic photoreceptor using the charge production material, being highly sensitive and highly resistant, and having a high image quality. Therefore, the application is totally different in purpose from the present invention, which intends to provide a material that has a high solubility in an organic solvent so as to be capable of being subjected to a solution process and has a high molecular orientation so as to be highly functional as a material of an organic FET element and the like.

That is to say, the invention of the application does not have such an object that is to increase solubility in an organic solvent. Therefore, the invention of the application does not have at all a technical idea to introduce a functional group such as an alkyl group into position 2 of a skeleton of the thiophene. In regard to the porphyrazine derivative in accordance with the present invention, it is necessary to use a compound represented by the general formula (8) as an intermediate as described in the section <2>, in order to introduce a functional group such as an alkyl group into position 2 of the skeleton of the thiophene. Further, in order to produce the compound represented by the general formula (8), it is necessary to transfer halogen from position 2 to position 3 of a compound represented by the general formula (9) and then cause halogenation at position 2 of the compound represented by the general formula (9) as described in the section <2>. The application does not disclose or suggest at all a method having such a feature. Therefore, even persons skilled in the art could not have been accomplished the present invention in view of the application.

### <3. Use in accordance with the present invention>

As described above, the compound in accordance with the present invention is uniquely characterized by having a high solubility in an organic solvent and having a high molecular orientation. Therefore, the compound in accordance with the present invention can be suitably used in an organic semiconductor electronic material and a device which is a photoelectric conversion element, a solar battery, a dye-sensitized solar cell element, an organic EL element, a liquid crystal display element, an organic thin-film transistor element, or a light-emitting device having an organic carrier transport layer.

The device in accordance with the present invention only has to use the compound and is not particularly limited in specific structure, material, size, shape, usage, and the like. However, because the compound in accordance with the present invention can be subjected to a solution process, the compound is particularly applicable to an electroinjection layer of an EL element, which has been extremely difficult to produce by the solution process, and an active layer of a solar battery. Therefore, the compound is expected to contribute to large reduction in production costs of the EL element and the solar battery.

Needless to say, the compound can be a useful electronic material because both a thiophene skeleton and porphyrazine have an excellent electronic carrier transport capacity.

The device in accordance with the present invention has a main subject not in individual examples specifically described in the present specification, but in providing a device that can be produced by a solution process and/or a novel device having a high functionality that is attributed to a high molecular orientation of the compound in accordance with the present invention. Therefore, it should be noted that apparatuses, components, and the like other than the specifically-described devices are also included in the scope of the present invention.

In the present invention, an organic thin-film transistor element is also called a field effect transistor (hereinafter may be referred to as "FET"). The organic thin-film transistor element is such that two electrodes (source electrode and drain electrode) are provided in contact with a semiconductor and an electric current between the electrodes is controlled by a voltage applied to another electrode that is called a gate electrode.

Generally, the organic thin-film transistor element has a structure (Metal-Insulator-Semiconductor; MIS structure) in which the gate electrode is electrically isolated by an insulative film. Such a structure that the insulative film is a metal oxide film is called an MOS structure. Apart from these, there is a structure in which the gate electrode is provided via a Schottky barrier, that is, an MES structure. In a case of an FET using an organic semiconductor material, the MIS structure is well used.

Embodiments of an organic thin-film transistor element of the present invention are further explained below with reference to drawings. However, the present invention is not limited to these embodiments.

Fig. 6 shows some embodiments of an organic thin-film transistor element in accordance with the present invention. In each of the embodiments, the reference sign 1 indicates a source electrode, 2 indicates a drain electrode, 3 indicates a gate electrode, 4 indicates a substrate, 5 indicates a dielectric layer, and 6 indicates an organic semiconductor material. It is possible to determine an arrangement of each layer and electrodes according to usage of the element. Each of elements shown in A through D, in which an electric current passes in a direction parallel to a substrate, is called a horizontal FET.

In Fig. 6, A shows an example of a bottom-contact type organic thin-film transistor element, and B shows an example of a top-contact or bottom-gate/top-contact type organic thin-film transistor element. Fig. 2 illustrates an FET element showing an embodiment of the top-contact type organic thin-film transistor element. In Fig. 2, "organic semiconductor layer" corresponds to "organic semiconductor material 6" shown in Fig. 6, likewise, "SiO₂ (200nm)" corresponds to "dielectric layer 5", and "n-Si" and "gate electrode (Au 50nm)" collectively correspond to "gate electrode 3" and "substrate 4".

In Fig. 6, C shows a structure well used in production of an organic single-crystal FET. In this structure, a source and drain electrodes and an insulative layer arc provided on a semiconductor and a gate electrode is formed thereon.

In Fig. 6, D shows a structure of a top & bottom-contact type transistor.

In Fig. 6, E shows a schematic view of an FET having a vertical structure, that is, a static induction transistor (SIT). In the SIT, a large amount of carrier can transfer at a time because an electric current runs in a plane. Further, a high-speed response can be achieved because a source electrode and a drain electrode are vertically provided so that a distance between the electrodes becomes small. Therefore, the SIT can be suitably used in high-current application, high-speed switching, and the like. In E of Fig. 6, no substrate is shown. However, substrates are normally provided outside a source and drain electrodes represented by the reference signs 1 and 2, respectively, in E of Fig. 6.

Next, components in each of the embodiments are described below. It is necessary that a substrate 4 can hold each layer to be formed on the substrate 4, so that the layers would not be taken off. For example, the substrate 4 may be made from an insulating material such as a resin plate, a film, paper, glass, quartz, and ceramic; an electrically conducting substrate, made from a metal or an alloy, on which an insulating layer is formed by coating or the like; or a material made from various combinations of a resin, an inorganic material, and the like.

The followings are examples of usable resin films: polyethylene terephthalate; polyethylene naphthalate; polyether sulfone; polyamide; polyimide; polycarbonate; cellulose triacetate; and polyetherimide. Use of a resin film or paper makes it possible to obtain a flexible organic thin-film transistor element. By this, it is possible obtain the device which is flexible and light in weight and is improved in practicality. The substrate is normally 1µm to 10mm, preferably 5µm to 5mm, in thickness.

Each of a source electrode 1, a drain electrode 2, and a gate electrode 3 is made from an electrically conducting material such as: a metal such as platinum, gold, silver, aluminum, chrome, tungsten, tantalum, nickel, cobalt, copper, iron, lead, tin, titanium, indium, palladium, molybdenum, magnesium, calcium, barium, lithium, potassium, and sodium, and an alloy including these metals; an electrically conductive oxide such as InO₂, ZnO₂, SnO₂ and ITO; an electrically conductive high polymer compound such as polyaniline, polypyrrole, polythiophene, polyacetylene, polyparaphenylene, vinylene, and polydiacetylene; semiconductors such as silicon, germanium, gallium arsenic; and a carbon material such as carbon black, fullerene, carbon nanotube, and graphite.

The electrically conductive high polymer compound and a semiconductor can be subjected to doping. In this case, a dopant may be, for example, an inorganic acid such as hydrochloric acid and sulfuric acid; an organic acid having an acid functional group, such as sulfonic acid; a Lewis acid such as PF₅, AsF₅, and FeCl₃; a halogen atom such as iodine; and a metallic atom such as lithium, sodium, and potassium. In a case of an inorganic semiconductor made from silicon or the like, boron, phosphorus, arsenicum, or the like is well used as the dopant. Alternatively, an electrically conductive composite material in which carbon black or metallic particles are dispersed in the dopant is also used.

As a determination factor of element characteristics, a distance (channel length) between a source electrode and a drain electrode is important. The channel length is normally 0.5µm to 500µm, preferably 0.5µm to 100µm. With a short channel length, it is possible to increase an amount of electric current to be taken out, however, there occurs a leakage electric current. Therefore, it is necessary to appropriately arrange the channel length.

It is difficult to categorically describe a width (channel width) between the source electrode and the drain electrode because the channel width is adjusted as appropriate according to a desired amount of electric current to be taken out. However, the channel length is normally 10µm to 5000µm, preferably 100µm to 3000µm. It is also possible to make the channel length longer than the range above with electrodes having an interleave structure or the like. The cannel length can be arranged as appropriate according to a desired amount of electric current, an element structure, and the like.

The following description deals with each structure (shape) of a source and drain electrodes. The source and drain electrodes may have a same structure or different structures from each other. Generally, in a case of a bottom-contact structure, it is preferable that each of the electrodes is formed by lithography and shaped into a rectangular parallelepiped.

It is possible that an electrode length is same as the channel width. There is no particular rule of an electrode width. However, for the purpose of reducing an element area, the electrode width is preferably small within such a range that an electric characteristic can be stabilized. The electrode width is normally 10µm to 5000µm, preferably 100µm to 3000µm. In addition, an electrode thickness is normally 1nm to 1µm, preferably 5nm to 0.5nm, more preferably 10nm to 0.2µm. Each of the electrodes 1, 2, and 3, is connected to a line. These lines are made from the substantially same material as the electrodes.

Used as a dielectric layer 5 is an insulating material such as: a polymer such as polyparaxylylene, polyacrylate, polymethyl methacrylate, polystyrene, polyvinylphenol, polyamide, polyimide, polycarbonate, polyester, polyvinyl alcohol, polyvinyl acetate, polyurethane, polysulphone, epoxy resin, and phenol resin, and a copolymer of these polymers; a metal oxide such as silica dioxide, aluminium oxide, titanium oxide, and tantalum oxide; a ferroelectric metal oxide such as SrTiO₃ and BaTiO₃; nitride such as silicon nitride and aluminium nitride; sulfide; a dielectric substance such as fluoride; or a polymer in which particles of the dielectric substance are dispersed. A thickness of an insulating layer 4 varies according to materials and is normally 0.1nm to 100µm, preferably 0.5nm to 50µm, more preferably 1nm to 10µm. How to form the dielectric layer 5 is described above.

As described above, the compound in accordance with the present invention can be used as an organic semiconductor material 6. The organic semiconductor material layer has a thickness of normally 1nm to 10µm, preferably 5nm to 5µm, more preferably 10nm to 3µm.

In the organic thin-film transistor element of the present invention, it is possible to provide other layers if necessary, for example, between a substrate and a dielectric layer, between the dielectric layer and an organic semiconductor material layer, and/or on an outer surface of the device. For example, a protection layer protects a semiconductor layer by removing external factors such as water and oxygen that cause degradation of an FET element characteristic. With the protection layer being provided on the semiconductor layer directly or via other layer(s), it is possible to reduce external air influence such as humidity and to increase an on/off ratio of the device. This provides an advantage in stabilization of an electric characteristic.

The protection layer is not particularly limited in material. However, it is preferable to use, for example: a resin film made from an epoxy resin, polyurethane, polyimide, polyvinylalcohol, a fluorine resin, polyolefin, an acryl resin such as polymethylmethacrylate, or the like; an inorganic oxide layer such as silicon oxide, aluminium oxide, and silicon nitride; and a film made from a dielectric substance, such as a nitride film. It is particularly preferable to use a resin (polymer) which is low in water absorbance and in transmittance of oxygen and water. Further, it is also possible to use a protection material which has been recently developed for an organic EL display. The protection layer can have any thickness according to its purpose, but normally has a thickness of 100nm to 1mm.

In the embodiments above, a substrate layer and a dielectric layer, or the dielectric layer and an organic semiconductor material layer, for example, can be accordingly formed by a vacuum deposition method, a spattering method, a coating method, a printing method, a sol-gel method, or the like.

Next, a production method of the organic thin-film transistor element in accordance with the present invention is described below with reference to Fig. 7 using as an example a bottom-contact type organic thin-film transistor element shown in A of Fig. 6.

This production method is also applicable to organic thin-film transistor elements of other embodiments.

Fig. 7 is a schematic view illustrating a production method of a bottom-contact type organic thin-film transistor element shown in A of Fig. 6.

### (Substrate and substrate treatment)

The organic thin-film transistor element of the present invention is produced by providing necessary layers and electrodes on a substrate 4 (see (1) of Fig. 7). The substrate is described above and may be arranged if necessary so as to have an electrode function.

### (Formation of gate electrode)

On the substrate 4, a gate electrode 3 is formed (see (2) of Fig. 7). The gate electrode is made from a material described above. As a method for forming an electrode film, it is possible to employ various methods such as a vacuum deposition method, a spattering method, a coating method, a thermal transfer method, a printing method, and a sol-gel method.

At the time of film formation or after the film formation, it is preferable to carry out patterning according to need so as to form a desired shape. It is possible to use various methods as a patterning method. For example, it is possible to use photolithography, which is a combination of photoresist patterning and etching. Further, it is also possible to carry out patterning by using a printing method such as inkjet printing, screen printing, offset printing, and letterpress, a technique of soft lithography such as a microcontact printing method, or a combination of a plurality of these techniques.

The gate electrode 3 has a thickness of normally 0.1nm to 10µm, preferably 0.5nm to 5µm, more preferably 1nm to 3µm, however, the thickness varies according to materials. In a case where the substrate doubles as the gate electrode, the thickness may be larger than the range above.

### (Formation of source electrode and drain electrode)

On the gate electrode 3, a dielectric layer 5 is formed (see (3) of Fig. 7). The dielectric layer is made from a material described above or the like. It is possible to use various methods for forming the dielectric layer. For example, it is possible to use: a coating method such as spin coating, spray coating, dip coating, cast, bar coating, and blade coating; a printing method such as screen printing, offset printing, and inkjet printing; and a dry process method such as a vacuum deposition method, molecular beam epitaxy, an ion cluster beam method, an ion plating method, a spattering method, an atmospheric pressure plasma method, and a CVD method. Apart from these, it is possible to use a sol-gel method and a method for forming an oxide film on a metal such as cases of forming alumite on aluminium and forming silicon dioxide on silicon.

### (Formation of source electrode and drain electrode)

It is possible to form a source electrode 1 and a drain electrode 2 by a method for forming a gate electrode 3 or according to the method (see (4) of Fig. 7).

### (Formation of organic semiconductor material layer)

As described above, the compound in accordance with the present invention can be used as an organic semiconductor material. As a method for forming an organic semiconductor material 6 on a dielectric layer 5, it is possible to accordingly employ, for example, a vacuum deposition method, a spattering method, a coating method, a printing method, a sol-gel method, or the like as described above (see (5) of Fig. 7). As described later in Examples, the compound in accordance with the present invention exhibits a high carrier mobility (p-type), a high on/off ratio, and an excellent threshold voltage. Therefore, the compound in accordance with the present invention is considered to have a high potential as the organic semiconductor material.

### (Formation of protection layer)

As described above, formation of a protection layer 7 on an organic semiconductor material layer provides such advantages that influence of external air can be reduced as small as possible and that an electric characteristic of an organic thin-film transistor element can be stabilized (see (6) of Fig. 7). It is possible to use, for the protection layer, a material described above.

It is possible to employ various methods for forming the protection layer. In a case where the protection layer is made from a resin, it is possible to use, for example: a method for coating with a resin solution and then drying the resin solution so that a resin film is formed; or a method for coating with or depositing resin monomers and then polymerizing the resin monomers. After film formation, a cross-linking treatment can be applied. In a case where the protection layer is made from an inorganic substance, it is possible to use, for example: a formation method with a vacuum process such as a spattering method and a deposition method; or a formation method with a solution process such as a sol-gel method.

In the organic thin-film transistor element of the present invention, the protection layer can be provided not only on the organic semiconductor material layer but also between other layers if necessary. These protection layers also may serve to stabilize an electric characteristic of the organic thin-film transistor element.

Concrete examples of the embodiments of the present invention are described below. The present invention is not limited to the examples and details of the present invention can vary in many ways. The present invention is not limited to the description of the embodiments above, but may be altered within the scope of the claims. An embodiment based on a proper combination of technical means disclosed in different embodiments is encompassed in the technical scope of the present invention.

### Examples

### Example 1

### (1. Production scheme: 1)

Fig. 1 shows a production scheme of an example of the compound in accordance with the present invention. Each reaction is described below in detail.

### [1] 2-Hexylthiophene (compound indicated by "1" in Fig. 1)

Under a nitrogen atmosphere, thiophene (10ml, 125mmol) was dissolved in THF (50mL) in a 300ml three-neck flask having a dropping funnel. After the resulting solution was cooled down to -78°C, n-BuLi (78.6mL) was added thereto in drops over 40 minutes. Then, t-BuOK (14g, 125mmol) was added thereto and then stirred for 1 hour at -78°C. To the resulting solution, 1-bromohexane (17.5ml, 125mmol) was added in drops over 30 minutes at -78°C. Then, the solution was stirred for 1.5 hours while the temperature was maintained. The reaction solution was slowly heated to a room temperature and stirred over night. Water and hexane (100mL) were added to the solution so that a water layer could be separated. The water layer was extracted with hexane (100mL×2). Then, organic layers were collected and sequentially washed with water (150mL), a salt solution (150mL), and water (150mLx3). The resulting solution was dried with anhydrous magnesium sulfate and thereafter filtered. Then, a solvent was removed therefrom under a reduced pressure. The residue thus obtained was subjected to column chromatography (activated silica gel, methylene chloride : hexane = 1:1), and then purified by bulb-to-bulb distillation under a reduced pressure. As a result, a compound indicated by "1" was obtained in the form of colorless liquid (15.7g, 55%).

TLC R_{f}=0.84 (silica gel, methylene chloride : hexane = 1:1); ¹H-NMR (400MHz, CDCl₃, TMS) ™7.10 (1H, dd, J=5.2Hz and 1.2Hz, position 5), 6.91 (1H, dd, J=5.2Hz and 3.6Hz, position 4), 6.77 (1H, dd, J=3.6Hz, and 1.2Hz, position 3), 2.82 (2H, t, J=8.2Hz), 1.67 (2H, m), 1.35 (6H, m), 0.89 (3H, t, J=7.0Hz); MS (70eV, EI) m/z=168 (M⁺)

### [2] 2-Bromo-5-hexylthiophene (compound indicated by "2" in Fig. 1)

In a 300mL three-neck flask having a dropping funnel, 2-hexylthiophene (compound indicated by "1") (6.2g, 37mmol) was dissolved in DMF (150mL). Then, a solution obtained by dissolving NBS (6.56g, 37mmol) in DMF (15mL) was added thereto in drops at a room temperature over 10 minutes and thereafter stirred for 12 hours at a room temperature. To the reaction solution thus obtained, a saturated sodium hydrogen carbonate solution (150mL) was added and then stirred for 30 minutes. Then, hexane (80mL) was added thereto. Subsequently, a water layer was separated and then extracted with hexane (80mL×2). Then, organic layers were collected, washed with water (100mL×3), and dried with anhydrous magnesium sulfate. After the resulting solution was filtered, a solvent was removed therefrom under a reduced pressure. As a result, a compound indicated by "2" was obtained in the form of yellow liquid (8.9g, 98%).

TLC R_{f}=0.79 (silica gel, methylene chloride: hexane = 1:1); ¹H-NMR (400MHz, CDCl₃, TMS) ™6.84 (1H, d, J=3.6Hz, position 3), 6.53 (1H, dt, J=3.6 and 1.2Hz, position 4), 2.74 (2H, t, J=7.6Hz), 1.62 (2H, m), 1.33 (6H, m), 0.88 (3H, t, J=7.2Hz); MS (70eV, EI) m/z=248 (M+).

### [3] 3-Bromo-5-hexylthiophene (compound indicated by "3" in Fig. 1)

Under a nitrogen atmosphere, 2-bromo-5-hexylthiophene (compound indicated by "2") (0.40g, 1.62mmol) was dissolved in THF (20mL) in a 50mL round bottom flask. After the resulting solution was cooled down to -60°C, 0.49M LDA-THF solution (6.56mL, 3.24mmol) was added thereto and then stirred for 2 hours while the temperature was maintained. Methanol (30mL) was added thereto and then stirred for 1 hour while the temperature was kept at -60°C. The resulting solution was slowly heated to a room temperature. Then, water (50mL) and hexane (50mL) was added so that a water layer was separated. The water layer was extracted with hexane (50mL×2). Then, organic layers were collected, washed with water (100mL×3), and dried with anhydrous magnesium sulfate. After the resulting solution was filtered, a solvent was removed therefrom under a reduced pressure. As a result, a compound indicated by "3" was obtained in the form of yellow liquid (391mg, 98%).

TLC R_{f}=0.79 (silica gel, methylene chloride : hexane = 1:1); ¹H-NMR (400MHz, CDCl₃, TMS) TM7.00 (1H, d, J=1.6Hz, position 2), 6.70 (1H, dt, J=1.6 and 1.2Hz, position 4), 2.77 (2H, t, J=7.6Hz), 1.65 (2H, m), 1.34 (6H, m), 0.88 (3H, t, J=7.2Hz); MS (70eV, EI) m/z=248 (M⁺).

### [4] 2,3-Dibromo-5-hexylthiophene (compound indicated by "4" in Fig. 1)

In a 50mL three-neck flask having a dropping funnel, 3-bromo-5-hexylthiophene (1.0g, 4.1mmol) was dissolved in DMF (36mL). Then, a solution obtained by dissolving NBS (720mg, 4.1mmol) in DMF (4mL) was added thereto in drops over 5 minutes, and thereafter stirred for 12 hours at a room temperature. To the resulting solution, a saturated sodium hydrogen carbonate solution (60mL) was added, and then stirred for approximately 30 minutes. Hexane (100mL) was added thereto. Subsequently, a water layer was separated and then extracted with hexane (100mL×2). Then, organic layers were collected, washed with water (100mL×3), dried with anhydrous magnesium sulfate, and thereafter filtered. Then, a solvent was removed from the resulting solution under a reduced pressure. The residue thus obtained was subjected to column chromatography (activated silica gel, methylene chloride : hexane = 1:1) so that an original component was removed. As a result, a compound indicated by "4" was obtained in the form of yellow liquid (1.32g, 93%).

TLC R_{f}=0.86 (silica gel, methylene chloride : hexane = 1:1); ¹H-NMR (400MHz, CDCl₃, TMS) ™7.04 (1H, t, J=0.8Hz, position 4), 2.87 (2H, t, J=7.2Hz), 1.70 (2H, m), 1.35 (6H, m), 0.89 (3H, t, J=6.8Hz); MS (70eV, EI) m/z=326 (M⁺)

### [5] 2,3-Dicyano-5-hexylthiophene (compound indicated by "5" in Fig. 1)

Under a nitrogen atmosphere, 2,3-dibromo-5-hexylthiophene (compound indicated by "4") (300mg, 0.912mmol) was dissolved in NMP (8mL) in a 50mL three-neck flask. Copper(I) cyanide (260mg, 2.92mmol) was added thereto and then heated to reflux at 210°C for 6 hours. To the resulting solution, a mixed solution of iron(III) chloride (1.04g, 6.44mmol) and 2M hydrochloric acid (20mL) was added, and then stirred for 30 minutes while being kept at 60°C to 70°C. After being cooled down to a room temperature, the mixed solution thus obtained was added with methylene chloride (60mL). Then, a water layer was separated and thereafter extracted with methylene chloride (60mL×2). After that, organic layers were collected and sequentially washed with 2M hydrochloric acid (100mL×2), water (100mL), a saturated sodium hydrogen carbonate solution (100mL), water (100mL), a salt solution (150mL×2), and water (100mL). Then, the resulting solution was dried with anhydrous magnesium sulfate and filtered, and thereafter a solvent was removed therefrom. The residue thus obtained was subjected to column chromatography (activated silica gel, methylene chloride : hexane = 1:1) so as to be purified. As a result, a compound indicated by "5" was obtained in the form of light green liquid (258mg, 67%).

TLC R_{f}=0.24 (silica gel, methylene chloride : hexane = 1:1); IR (KBr) 2222cm⁻¹; ¹H-NMR (400MHz, CDCl₃, TMS); TM7.04 (1H, t, J=0.8Hz, position 4), 2.87 (2H, t, J=7.2Hz), 1.70 (2H, m), 1.35 (6H, m), 0.89 (3H, t, J=6.8Hz); MS (70eV, EI) m/z=218 (M⁺).

### [6] Tetra(hexylthieno)porphyrazine (compound indicated by "6" in Fig. 1)

Under a nitrogen atmosphere, 2,3-dicyano-5-hexylthiophene (600mg, 2.75mmol) was dissolved in 1-pentanol (10mL) in a 50mL three-neck flask and heated to 100°C. Added thereto was 2.6M lithium 1-pentanoxide/1-pentanol solution (10.58mL, 27.5mmol) having been heated to 80°C. The reaction solution thus obtained was heated and stirred for 11 hours at 130°C. After a reaction, the solution was cooled down to a room temperature. Then, acetic acid (50mL) was added thereto and stirred for 30 minutes. After a solvent was removed from the solution under a reduced pressure, methanol was added so that reprecipitation occurred. The precipitation thus obtained was subjected to filtration and then washed with methanol (300mL). The resultant obtained by the filtration was subjected to column chromatography (alumina, methylene chloride), so that a compound indicated by "6" was obtained in the form of a purple solid substance (145mg, 24%).

Melting point 244°C to 245°C; TLC R_{f}=0.72 (silica gel, methylene chloride); IR (KBr) 3293cm⁻¹; ¹H-NMR (400MHZ, CDCl₃, TMS); ™7.6-7.8 (4H, m), 3.28 (8H, t, J=6.4Hz), 1.68 (32H, m), 1.03 (12H, t, J=6.8Hz), -4.70 (2H, brs); MS (MALDI-TOF, dithranol) m/z=857.6 (M+H⁺)

### [7] Copper (II) complex (compound indicated by "7" in Fig. 1)

Under a nitrogen atmosphere, a compound indicated by "6" (10mg, 0.0114mmol) and copper acetate (24mg, 0.133mmol) were dissolved in 1-pentanol (5mL) in a 50mL round bottom flask. The resulting solution was heated to reflux for 1 hour and then cooled down to a room temperature. Then, a solvent was removed therefrom under a reduced pressure. The residue thus obtained was subjected to column chromatography (alumina, methylene chloride) and recrystallized from toluene-ethyl acetate. As a result, a compound indicated by "7" was obtained in the form of green powder (5.3mg, 50%).

Melting point >300°C; TLC R_{f}=0.43 (alumina, methylene chloride); MS (MALDI, dithranol) m/z=936.8 (M+H⁺).

Note that operations which are not referred in the explanations above were carried out based on related literatures (D. W. MacDowell et al., J. Org. Chem. 1977, 42, 3717; M. J. Cook et al., J. Mater. Chem. 1997, 7, 5; M. J. Cook et al., Tetrahedron. 2000, 56, 4085; D. M. Knawby et al., Chem. Mater. 1997, 9, 535; M. Victoria Martinez-Diaz et al., Tetrahedron Lett. 2003, 44, 8475).

### (2. Solubility of respective compounds indicated by "6" and "7")

Respective compounds indicated by "6" and "7" were subjected to measurements of solubility in chloroform and in dichloromethane. In the measurements, approximately 3mg of a compound was prepared in advance by a precise weighing. Then, a solvent was added by 0.1mL to the compound and thereafter stirred or subjected to ultrasonic agitation. The solubility (mg/mL) was determined from an amount of the solvent used in dissolving a whole amount of the compound.

The compound 6 indicated by "6" in Fig. 1 had a solubility of 29mg/mL in chloroform and a solubility of 18mg/mL in dichloromethane.

The compound 7 indicated by "7" in Fig. 1 had a solubility of 11mg/mL in chloroform and a solubility of 10mg/mL in dichloromethane.

In contrast, an unsubstituted phthalocyanine derivative, which does not include an alkyl group, had a solubility of less than 0.02mg/mL in chloroform or dichloromethane. The unsubstituted phthalocyanine derivative is represented by the following structural formula:

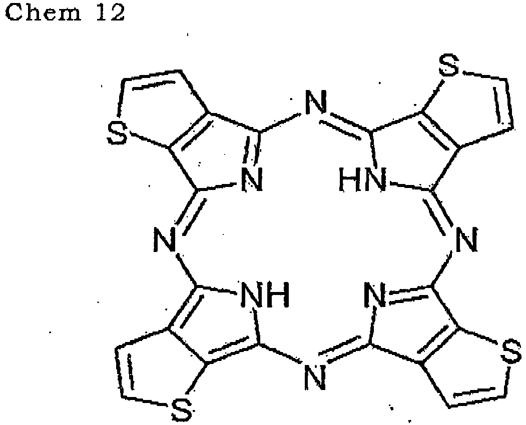

### (3. FET characteristic evaluation)

With use of the compound indicated by "6", a bottom-gate/top-contact type element was prepared by spin coating. Then, FET characteristics of the element were evaluated. Fig. 2 is a schematic view illustrating a structure of an FET element prepared by using the compound indicated by "6". Note that spin coating is a method for forming a thin film by dropping a solution onto a substrate and thereafter removing a solvent by spinning the substrate at a high speed.

As shown in Fig. 2, the FET element has a bottom-gate/top-contact type structure in which a source and drain electrodes are provided on an organic thin film. The FET element was produced in such a way that, on a silicon substrate that includes SiO₂ (having a thickness of 200nm) as an insulating layer, a film was formed as an organic semiconductor layer by spin coating with the compound indicated by "6". The FET element was then transferred into a vacuum apparatus, and gold was vacuum-deposited onto the element so that the source and drain electrodes each having a thickness of 50nm were formed. In this case, a channel length (interval between the source and drain electrodes) and a channel width (effective range of the electrodes) were 50µm and 1.5mm, respectively.

The following three types of elements were prepared: an element in which a surface of SiO₂ was treated with octyltrichlorosilane (OTS); an element in which a surface of SiO₂ was treated with hexamethyldisilazane (HMDS); and an untreated element. FET characteristic evaluation was carried out on respective elements which were, after production of the elements, not subjected to annealing, subjected to annealing at 100°C, and subjected to annealing at 120°C. The OTS and HMDS treatments are for controlling molecular orientation of an organic semiconductor layer.

Table 1 shows results of FET characteristic evaluation of respective FET elements that are treated/untreated with OTS and are/aren't subjected to annealing. In addition, Table 1 shows results of FET characteristic evaluation of respective FET elements that are treated with HMDS and are/aren't subjected to annealing.

The element that is untreated with OTS exhibited no FET response before annealing, but exhibited a carrier mobility (p-type) of over 10⁻³cm²/Vs in the atmosphere after annealing.

The element that is treated with OTS exhibited a carrier mobility (p-type) of over 10.³cm²/Vs in the atmosphere before annealing, and improved after annealing to exhibit a carrier mobility (p-type) of over 10⁻²cm²/Vs in the atmosphere. Further, in the element that is treated with OTS and subjected to annealing, an on/off ratio was improved to 5×10³. Therefore, in a case where the element that is treated with OTS and subjected to annealing is used in switching, it is possible to turn on/off an electric current in a circuit with a smaller voltage change than a case of the element that is untreated with OTS or the element that is either treated with OTS or subjected to annealing.

The element that is treated with HMDS exhibited a carrier mobility (p-type) of over 10⁻²cm²/Vs in the atmosphere even before annealing, and improved after annealing to exhibit a carrier mobility (p-type) of 10⁻¹cm²/Vs in the atmosphere. Further, in the element that is treated with HMDS and subjected to annealing, an on/off ratio was improved to 10⁵. As a result, it was found that the element that is treated with HMDS and subjected to annealing achieves a high-speed operation and makes it possible to turn on/off an electric current in a circuit with an extremely small change in voltage.

Note that annealing is such a treatment that a material is heated to below its melting point (decomposition point) and thereafter slowly and gradually cooled down. It is possible to carry out annealing by a known method. For example, it is preferable to use a method in which the organic semiconductor material is subjected to a treatment for 1 hour at not less than 80°C and not more than 200°C in a batch annealing oven whose heat source is gas, oil or electricity.

The OTS treatment can be such that a silicon substrate is immersed in a toluene solution of 0.1M OTS at 60°C for 20 minutes. The HMDS treatment can be such that a silicon substrate is exposed to HMDS vapor at a room temperature for 12 hours. Both of the OTS and HMDS methods have been known and were carried out based on related literatures (B. S. Ong et al., J. Am. Chem. Soc., 2004, 126, 3378; M. -H. Yoon et al., J. Am. Chem. Soc., 2005, 127, 1348).

In Table 1, Vₜₕ (V) means a threshold voltage that causes a drain electric current to start passing, and is preferably about OV.

**Table 1**

| | µ_{FET} (cm²V⁻¹s⁻¹) | Iₒₙ/I_{off} | Vₜₕ (V) |
|---|---|---|---|
| Untreated with OTS No annealing | No FET response | | |
| Untreated with OTS Annealing at 100°C | 1.10×10⁻³ | 10³ | -7.8 |
| Treated with OTS No annealing | 6.24×10⁻³ | 10³ | -14.4 |
| Treated with OTS Annealing at 100°C | 2.12×10⁻² | 5×10³ | -12.6 |
| Treated with HMDS No annealing | 1.51×10⁻² | 5×10² | -15.6 |
| Treated with HMDS Annealing at 1.20°C | 1.23×10⁻¹ | 10⁵ | -13.8 |

Fig. 3 is a graph showing FET characteristics of an element (i) produced with a silicon substrate having been treated with OTS and (ii) subjected to annealing at 100°C. In Fig. 3, (a) and (b) show an Id-Vg characteristic and an Id-Vd characteristic, respectively. As shown in (a) and (b) of Fig. 3, it turns out that this FET element exhibits typical p-type characteristics.

As described above, a substrate in which a surface of SiO₂ is untreated exhibited no FET response in a case where no annealing was applied, but became able to exhibit a carrier mobility of over 10⁻³cm²/Vs after annealing. This is because the compound in accordance with the present invention, that is, a porphyrazine derivative in which porphyrazine forms a condensed ring with thiophene and a skeleton of the thiophene has a functional group such as an alkyl group at position 2, has a high potential as an organic semiconductor layer.

Therefore, the compound in accordance with the present invention is highly useful as an organic semiconductor electronic material and in a device which is a photoelectric conversion element, a solar battery, a dye-sensitized solar cell element, an organic EL element, a liquid crystal display element, an organic thin-film transistor element, or a light-emitting device having an organic carrier transport layer.

In a case where an OTS treatment was applied, an increase in carrier mobility by 1 order was achieved due to annealing. Therefore, it can be said that the OTS treatment and annealing allow the compound indicated by "6" to more efficiently realize a potential as an organic semiconductor layer. The OTS treatment and annealing do not always have to be carried out, however, when producing an FET element, it is preferable to carry out either of them, and it is more preferable to carry out both of them.

Fig. 4 is a graph showing FET characteristics of an element (i) produced with a silicon substrate having been treated with HMDS and (ii) subjected to annealing at 120°C. In Fig. 4, (a) and (b) show an Id-Vg characteristic and an Id-Vd characteristic, respectively. As shown in (a) and (b) of Fig. 4, it turns out that this FET element exhibits typical p-type characteristics.

In Figs. 3 and 4, Id is an electric current value (A) of a drain electrode, and Vd and Vg are a voltage of the drain electrode and that of a gate electrode, respectively. Further, (-Id)^{1/2} is a square root of the electric current value (Id).

### Example 2

### (1. Production scheme: 2)

Fig. 5 shows a production scheme of another example of the compound in accordance with the present invention. Each reaction is described below in detail.

### [1] 2-Bromo-5-ethylthiophene (compound indicated by "1" in Fig. 5)

In a 200mL three-neck flask, 2-ethylthiophene (5g, 0.045mol) was dissolved in DMF (50mL). Then, a DMF solution (20mL) of NBS (7.93g, 0.045mol) was added thereto in drops and thereafter stirred at a room temperature for 12 hours. To the resulting solution, a saturated sodium hydrogen carbonate solution (150mL) was added, and then stirred for approximately 30 minutes. Then, a water layer was extracted with hexane (150mL×3), and an organic layer was washed with water (100mL×3). The resulting solution was dried with anhydrous magnesium sulfate, and a solvent was removed therefrom under a reduced pressure. As a result, a compound indicated by "1" was obtained in the form of yellow liquid (4.19g, 49%).
¹H-NMR (400MHz, CDCl₃, TMS) δ6.85 (1H, d, J=3.6Hz, position 3), 6.55 (1H, dt, J=3.6 and 1.2Hz, position 4), 2.78 (2H, quart, J=7.6Hz), 1.278 (3H, t, J=7.6Hz)

### [2] 3-Bromo-5-ethylthiophene (compound indicated by "2" in Fig. 5)

Under a nitrogen atmosphere, 2-bromo-5-ethylthiophene (compound indicated by "1") (4.0g, 0.021mol) was dissolved in THF (50mL) in a 300mL round bottom flask. After the resulting solution was cooled down to -60°C, 0.49M LDA-THF solution (82.6mL, 41mmol) was added thereto and then stirred for 2 hours while the temperature was maintained. Then, methanol (120mL) was added and stirred for 1 hour while the temperature was maintained. The resulting solution was gradually heated to a room temperature. After that, a water layer was extracted with hexane (200mL×3), and an organic layer was washed with water (400mL×3). The solution thus obtained was dried with anhydrous magnesium sulfate, and a solvent was removed therefrom under a reduced pressure. As a result, a compound indicated by "2" was obtained in the form of yellow liquid (3.33g, 83%).
¹H-NMR (400MHz, CDCl₃, TMS) δ7.00 (1H, d, J=1.2Hz, position 2), 6.71 (1H, dt, J=1.2Hz, position 4), 2.81 (2H, quart, J=7.4Hz), 1.29 (3H, t, J=7.8Hz)

### [3] 2,3-Dibromo-5-ethylthiophene (compound indicated by "3" in Fig. 5)

In a 100mL three-neck flask, 3-bromo-5-ethylthiophene (compound indicated by "2") (3.3g, 0.017mol) was dissolved in DMF (20mL). Then, a DMF solution (10mL) of NBS (3.0g, 0.017mol) was added thereto in drops and thereafter stirred for 10 hours at a room temperature. To the resulting solution, a saturated sodium hydrogen carbonate solution (50mL) was added, and then stirred for approximately 30 minutes. Then, a water layer was extracted with hexane (60mL×3), and an organic layer was sequentially washed with water (100mL×2), a salt solution (100mL), and water (100mL). The solution thus obtained was dried with anhydrous magnesium sulfate, and a solvent was removed therefrom under a reduced pressure. As a result, a compound indicated by "3" was obtained in the form of yellow liquid (4.3g, 92%).
¹H-NMR(400MHz, CDCl₃, TMS) δ6.62 (1H, t, J=1Hz, position 4), 2.76 (2H, quart, J=7.6Hz), 1.27 (3H, t, J=7.6Hz); MS (70cV, EI) m/z=270(M⁺)

### [4] 2,3-Dicyano-5-ethylthiophene (compound indicated by "4" in Fig. 5)

Under a nitrogen atmosphere, 2,3-dibromo-5-ethylthiophene (compound indicated by "3") (4.1g, 0.015mmol) and copper(I) cyanide (4.2g, 0.047mmol) were dissolved in NMP (50mL) in a 200mL three-neck flask. The resulting solution was heated to reflux at 210°C for 5 hours. Then, a mixed solution of FeCl₃ (16.6g, 0.102mol) and 2M hydrochloric acid (100mL) was added thereto and stirred for 30 minutes while being kept at 60°C to 70°C. After being cooled down, the mixed solution thus obtained was subjected to extraction using methylene chloride (200mL×3). Then, an organic layer was sequentially washed with 2M hydrochloric acid (250mL×2), water (300mL), a saturated sodium hydrogen carbonate solution (300mL), water (300mL), a salt solution (300mL×2), and water (300mL). The resulting solution was dried with anhydrous magnesium sulfate, and a solvent was removed therefrom under a reduced pressure. The residue thus obtained was subjected to column chromatography (activated silica gel, methylene chloride : hexane = 1:1) so as to be purified. As a result, a compound indicated by "4" was obtained in the form of a white granular crystal (1.09g, 48%). TLC R_{f}=0.15 (silica gel, methylene chloride : hexane = 1:1);
¹H-NMR (400MHz, CDCl₃, TMS) δ7.06 (1H, t, J=1Hz, position 4), 2.93 (2H, quart, J=7.8Hz), 1.38 (3H, t, J=7.8Hz); MS (70cV, EI) m/z=162 (M⁺)

### [5] Tetra(ethylthieno)porphyrazine (compound indicated by "5" in Fig. 5)

Under a nitrogen atmosphere, 2,3-dicyano-5-ethylthiophene (compound indicated by "4") (971mg, 5.99mmol) was dissolved in 1-pentanol (20mL) at 100°C in a 100mL round bottom flask. Then, 2.6M lithium 1-pentanoxide/ 1-pentanol solution (23mL, 59.9mmol) having been heated to 80°C was added thereto and stirred at 130°C for 10 hours. After being cooled down to a room temperature, the resulting solution was added with acetic acid (50mL) and stirred for 30 minutes. Then, a solvent was removed under a reduced pressure, and the residue thus obtained was added with methanol so that reprecipitation occurred. Then, a solid thus separated out was subjected to filtration and thereafter washed with methanol (300mL). The solid separated out was continuously subjected to extraction using chloroform. Then, a crystal thus separated out was subjected to filtration, so that a purple crystal (35mg) was obtained. Further, an extraction solution was concentrated and then added with methylene chloride-hexane so as to cause reprecipitation. As a result, a dark green solid (86mg) was obtained. In total, 121mg (12%) of a compound indicated by "5" was obtained.
MS (MALDI-TOF, dithranol) m/z=649.52 (M+H⁺)

### Example 3

### (1. Production scheme: 3)

Fig. 8 shows a production scheme of porphyrazine derivatives each having an alkyl group of a different length from one another, which porphyrazine derivatives serve as another examples of the compound in accordance with the present invention. As the alkyl group, an n-butyl group, an n-dodecyl group, or an n-hexadecyl group was used. Each reaction is described below in detail. Although Fig. 8 shows an production scheme of three different porphyrazine derivatives (Tetrakis(5-butylthiopheno)[2.3-b]porphyrazine, indicated by "1a" in Fig. 8; Tetrakis(5-dodecylthiopheno)[2.3-b]porphyrazine, indicated by "1b" in Fig. 8; and Tetrakis(5-cetylthiopheno)[2.3-b]porphyrazine, indicated by "1c" in Fig. 8), a same production method was used among these porphyrazine derivatives.

### [1] 2-Butylthiophene (compound indicated by "2a" in Fig. 8), 2-Dodecylthiophene (compound indicated by "2b" in Fig. 8), or 2-Cetylthiophene (compound indicated by "2c" in Fig. 8)

Under a nitrogen atmosphere, thiophene (5.6ml, 70mmol; 12.7ml, 159mmol; or 6ml, 75mmol) was dissolved in THF (50mL) in a 300mL three-neck flask having a dropping funnel. After the resulting solution was cooled down to -78°C, 1.6M n-BuLi (45mL, 100mL, or 48mL) was added thereto in drops over 40 minutes. Then, t-BuOK (7.8g, 70mmol; 17.8g, 159mmol; or 8.4g, 75mmol) was added and stirred for 1 hour at -78°C. Then, 1-butylhexane (7.5ml, 70mmol), 1-dodecylhexane (38.1ml, 159mmol), or 1-cetylhexane (23ml, 75mmol) was added in drops over 30 minutes and stirred for 1.5 hours while the temperature was maintained. The reaction solution thus obtained was slowly heated to a room temperature and stirred over night. To the solution, water and hexane (100mL) was added so that a water layer was separated. The water layer was extracted with hexane (100mL×2). Then, organic layers were collected and sequentially washed with water (150mL), a salt solution (150mL), and water (150mL×3). The resulting solution was dried with anhydrous magnesium sulfate and thereafter filtered. Then, a solvent was removed therefrom under a reduced pressure. The residue thus obtained was subjected to column chromatography (activated silica gel, methylene chloride : hexane = 1:1) and then purified by bulb-to-bulb distillation under a reduced pressure. As a result, a compound indicated by "2a" (6.4g, 64% yield), a compound indicated by "2b" (26g, 65% yield), and a compound indicated by "2c" (15.5g, 67% yield) were obtained respectively in the form of colorless liquid.

### (Data of 2-Butylthiophene)

¹H-NMR (CDCl₃) δ7.10 (1H, dd, J=5.2Hz, 1.2Hz), 6.91 (1H, dd, J=5.2Hz, 2.4Hz), 6.78-6.77 (1H, m), 2.82 (2H, t, J=8.0Hz), 1.66 (2H, quint, J-8.0Hz), 1.39 (2H, sext, J=8.0Hz), 0.93 (3H, t, J=7.6Hz); EI-MS, m/z=140 (M⁺)

### (Data of 2-Dodecylthiophene)

¹H-NMR (CDCl₃) δ7.10 (1H, dd, J=5.2Hz, 1,2Hz), 6.91(1H, dd, J=5.2Hz, 3.6Hz), 6.77 (1H, m), 2.81 (2H, t, J=7.6Hz), 1.66 (2H, quint, J=7.6Hz), 1.21-1.40 (18H, m), 0.88 (3H, t, J=7.2Hz); EI-MS, m/z=252 (M⁺)

### (Data of 2-Cetylthiophene)

¹H-NMR (CDCl₃) δ7.10 (1H, dd, J=5.2Hz, 1.2Hz), 6.91 (1H, dd, J=5.2Hz, 2.4Hz), 6.78-6.77 (1H, m), 2.82 (2H, t, J=8.0Hz), 1.66 (2H, quint, J=8.0Hz), 1.39 (2H, sext, J=8.0Hz), 0.93 (3H, t, J=7.6Hz); EI-MS, m/z=308 (M⁺)

### [2] 2-Bromo-5-butylthiophene (compound indicated by "3a" in Fig. 8), 2-Bromo-5-dodecylthiophene (compound indicated by "3b" in Fig. 8), or 2-Bromo-5-cetylthiophene (compound indicated by "3c" in Fig. 8)

In a 300mL three-neck flask having a dropping funnel, 2-butylthiophene (compound indicated by "2a") (6.0g, 42mmol), 2-dodecylthiophene (compound indicated by "2b") (20g, 79mmol), or 2-cetylthiophene (compound indicated by "2c") (13g, 45mmol) was dissolved in DMF (150mL).

Then, a solution obtained by dissolving NBS (7.6g, 42mmol; 14.1g, 79mmol; or 8.05g, 45mmol) in DMF (50mL) was added in drops at a room temperature over 10 minutes and stirred at a room temperature for 12 hours. The reaction solution thus obtained was added with a saturated sodium hydrogen carbonate solution (150mL), stirred for 30 minutes, and added with hexane (80mL). Then, a water layer separated was extracted with hexane (80mL×2). After that, organic layers were collected, washed with water (100mL×3), and dried with anhydrous sulfate magnesium. The resulting solution was filtered, and a solvent was removed therefrom under a reduced pressure. As a result, obtained was a compound indicated by "3a" (8.8g, 89% yield) which is in the form of liquid, a compound indicated by "3b" (24.8g, 95% yield) which is in the form of faint yellow liquid, or a compound indicated by "3c" (15.8g, 91% yield) which is in the form of a white solid.

### (Data of 2-Bromo-5-butylthiophene)

¹H-NMR (CDCl₃) δ6.60 (1H, t, J=0.8Hz), 2.82 (2H, t, J=8.0Hz), 1.66 (2H, quint, J=8.0Hz), 1.39 (2H, sext, J=8.0Hz), 0.93 (3H, t, J=7.6Hz); EI-MS, m/z=218 (M⁺)

### (Data of 2-Bromo-5-dodecylthiophene)

¹H-NMR (CDCl₃) δ6.84 (1H, d, J=3.6Hz), 6.53 (1H, dt, J=3.6Hz, 1.2Hz), 2.73 (2H, t, J=7.6Hz), 1.58-1.64 (2H, m), 1.20-1.35 (18H, m), 0.88 (3H, t, J=7.2Hz); EI-MS, m/z=332 (M⁺)

### (Data of 2-Bromo-5-cetylthiophene)

Melting point 31-32°C; ¹H-NMR (CDCl₃) δ6.84 (1H, d, J=3.6Hz), 6.52 (1H, d, J=3.6Hz), 2.73 (2H, t, J=8.0Hz), 1.62 (2H, quint, J=8.0Hz), 1.38-1.15 (26H, m), 0.87 (3H, t, J=7.2Hz); EI-MS, m/z=386 (M⁺)

### [3] 3-Bromo-5-butylthiophene (compound indicated by "4a" in Fig. 8), 3-Bromo-5-dodecylthiophene (compound indicated by "4b" in Fig. 8), 3-Bromo-5-cetylthiophene (compound indicated by "4c" in Fig. 8)

Under a nitrogen atmosphere, 2-bromo-5-butylthiophene (compound indicated by "3a") (8.0g, 37mmol), 2-bromo-5-dodecylthiophene butylthiophene (compound indicated by "3b") (7.0g, 21mmol), or 2-Bromo-5-cetylthiophene (compound indicated by "3c") (12g, 31mmol) was dissolved in THF (20mL) in a 50mL round bottom flask. After the resulting solution was cooled down to -60°C, 0.607M LDA-THF solution (60mL, 37mmol; 35mL, 21mmol; or 51mL, 31mmol) was added thereto and stirred for 2 hours while the temperature was maintained. Then, the resulting solution was added with methanol (30mL), stirred for 1 hour while being kept at -60°C, and then slowly heated to a room temperature. After water (50mL) and hexane (50mL) was added to the solution, a water layer was separated and thereafter extracted with hexane (50mL×2). Then, organic layers were collected, washed with water (100mL×3), and dried with anhydrous magnesium sulfate. The resulting solution was filtered, and a solvent was removed therefrom under a reduced pressure. As a result, obtained was a compound indicated by "4a" (7.5g, 93% yield) which is in the form of orange-colored liquid, a compound indicated by "4b" (6.5g, 93% yield) which is in the form of orange-colored liquid, or a compound indicated by "4c" (11.8g, 98% yield) which is in the form of orange-colored liquid.

### (Data of 3-bromo-5-butylthiophene)

¹H-NMR (CDCl₃) δ7.00 (1H, d, J=1.6Hz), 6.69 (1H, t, J=1.2Hz), 2.82 (2H, t, J=8.0Hz), 1.66 (2H, quint, J=8.0Hz), 1.39 (2H, sext, J=8.0Hz), 0.93 (3H, t, J=7.6Hz); EI-MS, m/z=218 (M⁺)

### (Data of 3-bromo-5-dodecylthiophene)

¹H-NMR (CDCl₃) δ7.00 (1H, d, J=1.6Hz), 6.69 (1H, t, J=1.2Hz), 2.76 (2H, t, J=7.6Hz), 1.64-1.60 (2H, m), 1.40-1.20 (18H, m), 0.88 (3H, t, J=7.2Hz); EI-MS, m/z=301 (M⁺)

### (Data of 3-bromo-5-cetylthiophene)

¹H-NMR (CDCl₃) δ7.00 (1H, d, J=1.6Hz), 6.69 (1H, t, J=1.2Hz), 2.73 (2H, t, J=8.0Hz), 1.62 (2H, quint, J=8.0Hz), 1.38-1.15 (26H, m), 0.87 (3H, t, J=7.2Hz); EI-MS, m/z=386 (M⁺)

### [4] 2,3-Dibromo-5-butylthiophene (compound indicated by "5a" in Fig. 8), 2,3-Dibromo-5-dodecylthiophene (compound indicated by "5b" in Fig. 8), or 2,3-Dibromo-5-cetylthiophene (compound indicated by "5c" in Fig. 8)

In a 50mL three-neck flask having a dropping funnel, 3-bromo-5-butylthiophene (6.8g, 31mmol), 3-bromo-5-dodecylthiophene (4.0g, 12mmol), or 3-bromo-5-cetylthiophene (10g, 26mmol) was dissolved in DMF (36mL). Then, NBS (5.5g, 31mmol; 2.2g, 12mmol; or 4.6g, 26mmol) dissolved in DMF (30mL) was added thereto in drops over 5 minutes and stirred for 12 hours at a room temperature. The resulting solution was added with a saturated sodium hydrogen carbonate solution (60mL), stirred for approximately 30 minutes, and then added with hexane (100mL). Then, a water layer was separated and thereafter extracted with hexane (100mL×2). After that, organic layers were collected, washed with water (100mL×3), and dried with anhydrous magnesium sulfate. The resulting solution was filtered, and a solvent was removed therefrom under a reduced pressure. The residue thus obtained was subjected to column chromatography (activated silica gel, methylene chloride : hexane = 1:1) so that an original component was removed. As a result, obtained was a compound indicated by "5a" (8.0g, 87% yield) which is in the form of faint yellow liquid, a compound indicated by "5b" (4.6g, 94% yield) which is in the form of faint yellow liquid, or a compound indicated by "5c" (10g, 83% yield) which is in the form of a white solid.

### (Data of 2,3-dibromo-5-butylthiophene)

¹H-NMR (CDCl₃) δ6.60 (1H, t, J=0.8Hz), 2.70 (2H, t, J=7.2Hz), 1.63-1.58 (2H, m), 1.38-1.17 (18H, m), 0.88 (3H, t, J=6.8Hz); EI-MS, m/z=406 (M⁺)

### (Data of 2,3-dibromo-5-dodecylthiophene)

¹H-NMR (CDCl₃) δ6.60 (1H, t, J=0.8Hz), 2.70 (2H, t, J=7.2Hz), 1.63-1.58 (2H, m), 1.38-1.17 (18H, m), 0.88 (3H, t, J=6.8Hz); EI-MS, m/z=406 (M⁺)

### (Data of 2,3-dibromo-5-cetylthiophene)

Melting point 31-32°C; ¹H-NMR (CDCl₃) δ6.60 (1H, t, J=0.8Hz), 2.73 (2H, t, J=8.0Hz), 1.62 (2H, quint, J=8.0Hz), 1.38-1.15 (26H, m), 0.87 (3H, t, J=7.2Hz); EI-MS, m/z=464 (M⁺)

### [5] 2,3-Dicyano-5-butylthiophene (compound indicated by "6a" in Fig. 8), 2,3-Dicyano-5-dodecylthiophene (compound indicated by "6b" in Fig. 8), or 2,3-Dicyano-5-cetylthiophene (compound indicated by "6c" in Fig. 8)

Under a nitrogen atmosphere, 2,3-dibromo-5-butylthiophene (compound indicated by "5a") (7.0g, 23mmol), 2,3-dibromo-5-dodecylthiophene butylthiophene (compound indicated by "5b") (3.5g, 12mmol), or 2,3-dibromo-5-cetylthiophene (compound indicated by "5c") (7.0g, 15mmol) was dissolved in NMP (60mL) in a 50mL threc-neck flask. Then, coppcr(I) cyanide (6.2g, 70mmol; 3.1g, 35mmol; or 4.0g, 45mmol) was added thereto and heated to reflux at 210°C for 6 hours. The resulting solution was added with a mixed solution of iron(III) chloride (1.04g, 6.44mmol) and 2M hydrochloric acid (20mL) and stirred for 30 minutes while being kept at 60°C to 70°C. After the mixed solution thus obtained was cooled down to a room temperature, methylene chloride (60mL) was added thereto. Then, a water layer was separated and thereafter extracted with methylene chloride (60mL×2). After that, organic layers were collected and sequentially washed with 2M hydrochloric acid (100mL×2), water (100mL), a saturated sodium hydrogen carbonate solution (100mL), water (100mL), a salt solution (150mL×2), and water (100mL). The resulting solution was dried with anhydrous magnesium sulfate and filtered, and a solvent was removed therefrom under a reduced pressure. The residue thus obtained was subjected to column chromatography (activated silica gel, methylene chloride : hcxane = 1:1) so as to be purified. As a result, obtained was a compound indicated by "6a" (3.5g, 80% yield) which is in the form of faint yellow liquid, a compound indicated by "6b" (1.7g, 66% yield) which is in the form of a yellow solid, or a compound indicated by "6c" (3.6g, 60% yield) which in the form of a white solid.

### (Data of 2,3-dicyano-5-butylthiophene)

¹H-NMR (CDCl₃) δ7.04 (1H, t, J=0.8Hz), 2.82 (2H, t, J=8.0Hz), 1.66 (2H, quint, J=8.0Hz), 1.39 (2H, sext, J=8.0Hz), 0.93 (3H, t, J=7.6Hz); IR (NaCl) 2223cm⁻¹; EI-MS, m/z=190 (M⁺)

### (Data of 2,3-dicyano-5-dodecylthiophene)

Melting point 33-34°C; ¹H-NMR (CDCl₃) δ7.04 (1H, t, J=0.8Hz), 2.87 (2H, t, J=7.6Hz), 1.70 (2H, quint, J=7.2Hz), 1.38-1.21 (18H, m), 0.89 (3H, t, J=7.2Hz); IR (KBr) 2222cm⁻¹; EI-MS, m/z=302 (M⁺)

### (Data of 2,3-dicyano-5-cetylthiophene)

Melting point 51-52°C; ¹H-NMR (CDCl₃) δ7.04 (1H, t, J=0.8Hz), 2.73 (2H, t, J=8.0Hz), 1.62 (2H, quint, J=8.0Hz), 1.38-1.15 (26H, m), 0.87 (3H, t, J=7.2Hz); IR (KBr) 2233cm⁻¹; EI-MS, m/z=358 (M⁺)

### [6] Tetrakis(5-butylthiopheno)[2.3-b]porphyrazine (compound indicated by "1a" in Fig. 8), Tetrakis (5-dodecylthiopheno)[2.3-b]porphyrazine (compound indicated by "1b" in Fig. 8), or Tetrakis(5-cetylthiopheno)[2.3-b]porphyrazine (compound indicated by "1c" in Fig. 8)

Under a nitrogen atmosphere, 2,3-dicyano-5-butylthiophene (1.0g, 5.3mmol), 2,3-dicyano-5-dodecylthiophene (410mg, 1.36mmol), or 2,3-dicyano-5-cetylthiophene (1.2g, 3.3mmol) was added to 2.6M lithium 1-pentanoxide/1-pentanol solution (20mL, 52.5mmol; 3mL, 7.8mmol; or 12.8mL, 33.4mmol) having been heated to 80°C in a 50mL three-neck flask. The reaction solution thus obtained was heated and stirred at 130°C for 2 hours.

After a reaction, the solution was cooled down to a room temperature, added with acetic acid (5mL), and stirred for 30 minutes. After a solvent was removed from the solution under a reduced pressure, methanol was added thereto so that reprecipitation occurred. The precipitation thus obtained was subjected to filtration and washed with methanol (300mL). The resultant obtained by the filtration was subjected to column chromatography (alumina, methylene chloride). As a result, obtained was a compound indicated by "1a" (147mg, 15% yield) which is in the form of a green solid, a compound indicated by "1b" (112mg, 27% yield) which is in the form of a green solid, or a compound indicated by "1c" (295mg, 24% yield) which is in the form of a green solid.

### (Data of tetrakis(5-butylthiopheno)[2.3-b]porphyrazine)

Melting point 280°C (dec); ¹H-NMR (CDCl₃) δ7.68-7.55 (4H, m), 2.10-1.95 (8H, m), 3.25-3.15 (8H, m), 1.73-1.62 (8H, m), 1.20-1.14 (12H, t, J=7.6Hz), -5.76 (2H, brs); IR (KBr) 3290cm⁻¹; EI-MS, m/z=763 (M⁺)

### (Data of tetrakis(5-dodecylthiopheno[2.3-b]porphyrazine)

Melting point 176-177°C; ¹H-NMR (CDCl₃) δ7.8-7.76 (4H, m), 3.29 (8H, t, J=7.6Hz), 1.67-1.28 (72H, m), 0.86 (12H, t, J=7.6Hz), -4.76 (2H, brs); IR (KBr) 3295cm⁻¹; MALDI-TOF-MS, m/z=1210 (M⁺)

### (Data of tetrakis(5-cetylthiopheno)[2.3-b]porphyrazine)

Melting point 151-152°C; ¹H-NMR (CDCl₃/CS₂) δ8.10-8.00 (4H, m), 3.40-3.29 (8H, m), 2.20-2.04 (8H, m), 1.73-1.61 (8H, m), 1.50-1.15 (96H, m), 0.88-0.85 (12H, m); IR (KBr) 3293cm⁻¹; MALDI-TOF-MS, m/z=1434 (M⁺+H)

The porphyrazine derivatives indicated by 1a through 1c, respectively, were identified based on their ¹H-NMR spectra and mass spectra. Figs. 9, 10, and 11 show NMR spectra of the porphyrazine derivative indicated by 1a (Tetrakis(5-butylthiopheno)[2.3-b]porphyrazine), the porphyrazine derivative indicated by 1b (Tetrakis(5-dodecylthiopheno)[2.3-b]porphyrazine), and the porphyrazine derivative indicated by 1c (Tetrakis(5-cetylthiopheno)[2.3-b]porphyrazine), respectively, each of which is dissolved in a chloroform-d solution.

### (2. FET characteristic evaluation of porphyrazine derivatives indicated by 1a and 1b, respectively)

On a silicon substrate including SiO₂ (200nm in thickness) as an insulating layer, an organic semiconductor layer was formed by spin coating with a porphyrazine derivative indicated by 1a or 1b. Thus, a bottom-gate/top-contact type FET element was formed so as to have the same structure as an element produced with a compound indicated by "6". The FET element was subjected to FET characteristic evaluation. Note that, in the FET element, a thickness of gold serving as a source and drain electrodes, a channel length, and a channel width were same as in the element produced with the compound indicated by "6".

The FET element was treated with OTS on a surface of SiO₂ in the same way as in Example 1. The FET characteristic evaluation was carried out on the FET element which was not subjected to annealing or was subjected to annealing at 100°C after production of the element.

Table 2 shows results of FET characteristic evaluation of the FET element which was/wasn't subjected to annealing. The evaluation was carried out in the atmosphere at a room temperature. The FET elements of both cases exhibited p-type semiconductor characteristics.

**Table 2**

| Compound | Surface treatment of SiO₂ substrate Annealing | µ_{FET} (cm²V⁻¹s⁻¹) | Iₒₙ/I_{off} | Vₜₕ (V) |
|---|---|---|---|---|
| 1a | OTS treatment No annealing | 1.65×10⁻⁴ | 10 | -15.8 |
| 1a | OTS treatment Annealing at 100°C | 4.75×10⁻⁴ | 10² | -18.5 |
| 1b | OTS treatment No annealing | 2.87×10⁻² | 10⁴ | -10.1 |
| 1b | OTS treatment Annealing at 100°C | 7.11×10⁻² | 5×10⁴ | -12.6 |

The porphyrazine derivative indicated by 1a exhibited a carrier mobility of over 10⁻⁴cm²/Vs and improved in carrier mobility and on/off ratio by annealing.

The porphyrazine derivative indicated by 1b exhibited a carrier mobility of over 10⁻²cm²/Vs and a high on/off ratio, i.e., 10⁴, even without annealing and further improved in these values by annealing. This indicates that the porphyrazinc derivative indicated by 1b achieves a high-speed operation and makes it possible to turn on/off an electric current in a circuit with a small change in voltage. In addition, the porphyrazine derivative indicated by 1b exhibited a low threshold voltage.

From the results shown in Tables 1 and 2, it can be assumed that the compound in accordance with the present invention can exhibit better FET characteristics when thiophene includes an alkyl group having a larger carbon number.

The compound, indicated by "5" in Fig. 5, in which thiophene includes an ethyl group as an alkyl group, has a low solubility in an organic solvent as is apparent from Example 2 [5] where the compound is continuously subjected to extraction using chloroform. This indicates that it is extremely difficult to form an organic semiconductor layer by, for example, spin coating using a chloroform solution of this compound. Therefore, also in a case of device production using a solution process, it is preferable to use a compound in which thiophene includes such an alkyl group that has a large carbon number, that is, a carbon number of approximately 4 or more.

The compound in accordance with the present invention is a novel compound, which is a porphyrazine derivative in which porphyrazine forms a condensed ring with thiophene and a skeleton of the thiophene has a functional group such as an alkyl group at position 2. Therefore, the compound in accordance with the present invention achieves a high solubility in an organic solvent and a high molecular orientation, and has a high potential as an organic semiconductor material. Because of this, the compound in accordance with the present invention is highly useful as a device material for a photoelectric conversion element, a solar battery, a dye-sensitized solar cell element, an organic EL element, a liquid crystal display element, an organic thin-film transistor element, or the like.

Also disclosed are a production method of the compound and of an intermediate occurred in the course of production of the compound.

The embodiments and concrete examples of implementation discussed in the foregoing detailed explanation serve solely to illustrate the technical details of the present invention, which should not be narrowly interpreted within the limits of such embodiments and concrete examples.

## Claims

1. Use of a compound as an organic semiconductor material, the compound being a porphyrazine derivative forming a condensed ring with thiophene having a skeleton
**characterized in that** the compound has on the thiophene skeleton in the 2-position an alkyl group being a C₁-C₃₀ alkyl group whose one or more carbon atoms may be substituted with an oxygen atom, an nitrogen atom, and/or a sulfur atom.

2. The use as set forth in claim 1, the compound being represented by the following general formula (1): 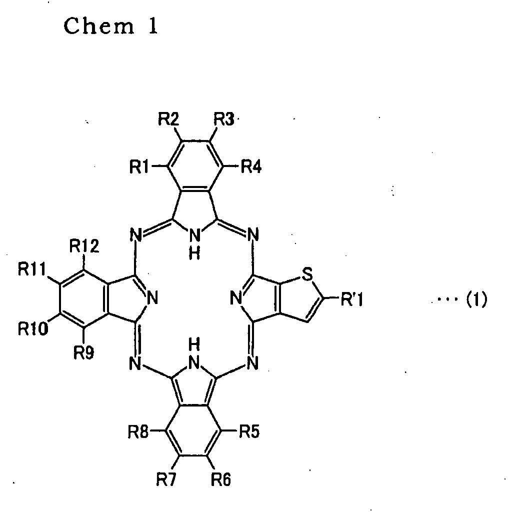 where:
R1 to R12 are independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
R'1 is an alkyl group, the alkyl group being a C₁-C₃₀ alkyl group whose one or more carbon atoms may be substituted with an oxygen atom, an nitrogen atom, and/or a sulfur atom.

3. The use as set forth in claim 1, the compound being represented by the following general formula (2): 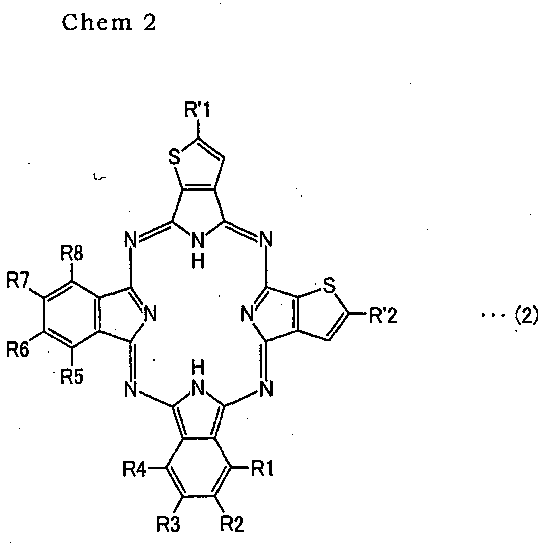 where:
R1 to R8 are independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
R'1 and R'2 are an alkyl group, the alkyl group being a C₁-C₃₀ alkyl group whose one or more carbon atoms may be substituted with an oxygen atom, an nitrogen atom, and/or a sulfur atom.

4. The useas set forth in claim 1, the compound being represented by the following general formula (3): 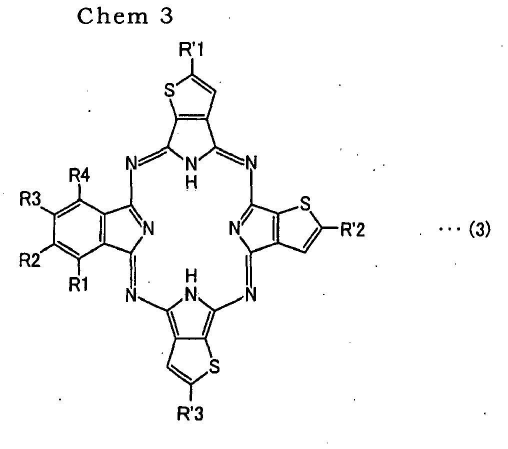
where R1 to R4 are independently a hydrogen atom, a halogen atom, an alkyl group, an alkoxy group, an alkylthio group, or an aromatic group, the alkyl group being a C₁-C₃₀ alkyl group whose one or more carbon atoms may be substituted with an oxygen atom, an nitrogen atom, and/or a sulfur atom, the alkoxy group being a C₁-C₃₀ alkoxy group whose one or more carbon atoms may be substituted with an oxygen atom, an nitrogen atom, and/or a sulfur atom, and the alkylthio group being a C₁-C₃₀ alkylthio group whose one or more carbon atoms may be substituted with an oxygen atom, an nitrogen atom, and/or a sulfur atom; and
R'1 to R'3 are an alkyl group, the alkyl group being a C₁-C₃₀ alkyl group whose one or more carbon atoms may be substituted with an oxygen atom, an nitrogen atom, and/or a sulfur atom,.

5. The use as set forth in claim 1, the compound being represented by the following general formula (4): 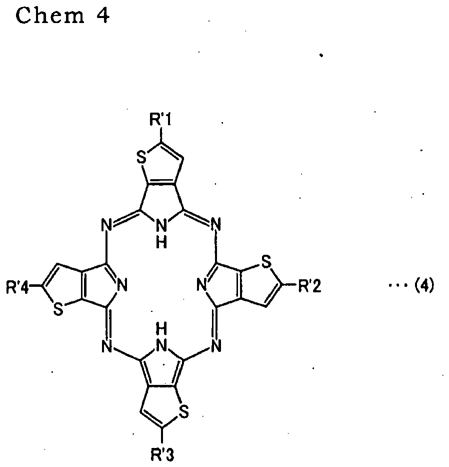 where R'1 to R'4 are an alkyl group, the alkyl group being a C₁-C₃₀ alkyl group whose one or more carbon atoms may be substituted with an oxygen atom, an nitrogen atom, and/or a sulfur atom.

6. The use as set forth in claim 1, the compound being represented by any one of the following chemical formula (5), (6), and (7): 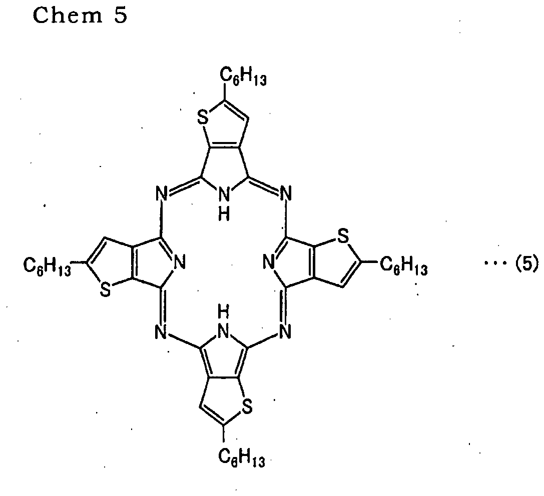 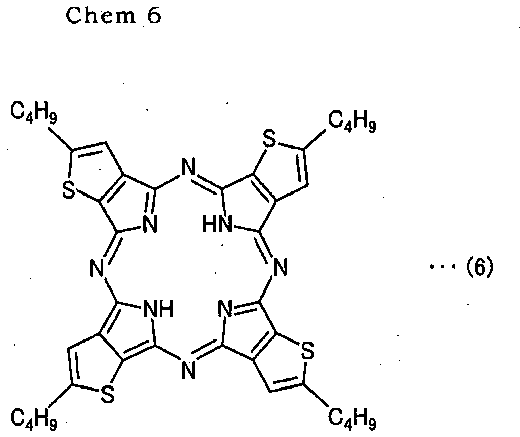 and

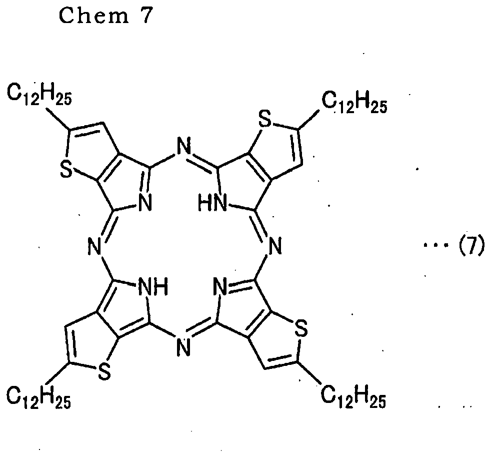

7. The use as set forth in any one of claims 1 to 6, wherein the porphyrazine of the compound forms a complex with a metal ion.

8. A device comprising a compound as set forth in any one of claims 1 to 7; the device being a photoelectric conversion element, a solar battery, a dye-sensitized solar cell element, an organic EL element, a liquid crystal display element, an organic thin-film transistor element, or a light-emitting device having an organic carrier transport layer.

9. The device as set forth in claim 8, wherein the organic thin-film transistor element is a top-contact type element.

10. The device as set forth in claim 8, wherein the organic thin-film transistor element is a bottom-contact type element.

## Patentansprüche

1. Verwendung einer Verbindung als ein organisches Halbleitermaterial, wobei die Verbindung ein Porphyrazin-Derivat ist, das einen kondensierten Ring mit Thiophen mit einem Skelett bildet, **dadurch gekennzeichnet, dass** die Verbindung an dem Thiophen-Skelett in der 2-Position eine Alkylgruppe hat, die eine C₁-C₃₀-Alkylgruppe ist, deren eines oder mehrere Kohlenstoffatome durch ein Sauerstoffatom, ein Stickstoffatom und/oder ein Schwefelatom substituiert sein kann/können.

2. Verwendung nach Anspruch 1, wobei die Verbindung durch die folgende allgemeine Formel (1) repräsentiert wird: 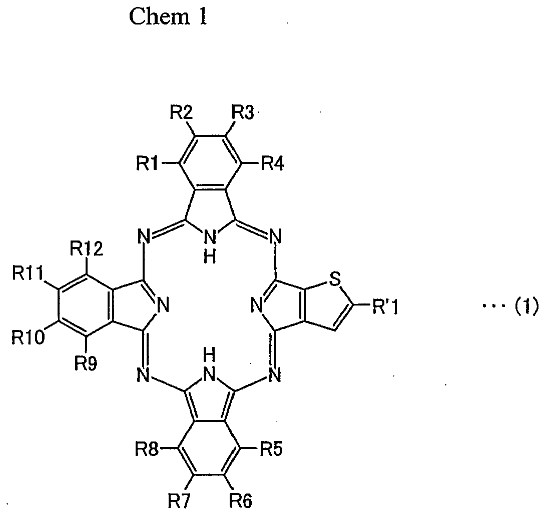 wobei:
R1 bis R12 unabhängig ein Wasserstoffatom, ein Halogenatom, eine Alkylgruppe, eine Alkoxygruppe, eine Alkylthiogruppe oder eine aromatische Gruppe sind, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können, wobei die Alkoxygruppe eine C₁-C₃₀-Alkoxygruppe ist, deren eines oder mehrere Kohlenstoffatome durch ein Sauerstoffatom, ein Stickstoffatom und/oder ein Schwefelatom substituiert sein können, und wobei die Alkylthiogruppe eine C₁-C₃₀-Alkylthiogruppe ist, deren eines oder mehrere Kohlenstoffatome durch ein Sauerstoffatom, ein Stickstoffatom und/oder ein Schwefelatom substituiert sein können; und
R'1 eine Alkylgruppe ist, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können.

3. Verwendung nach Anspruch 1, wobei die Verbindung durch die folgende allgemeine Formel (2) dargestellt wird: 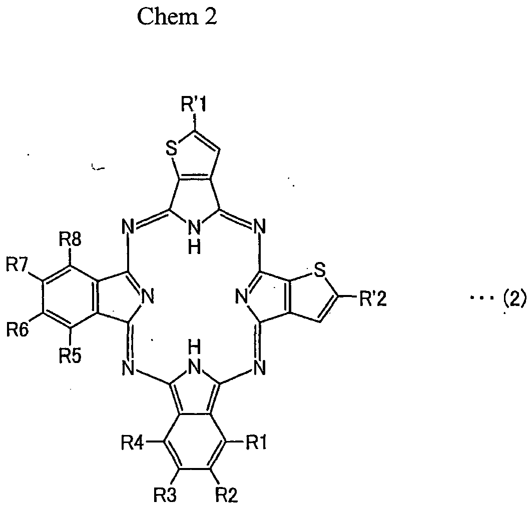 wobei:
R1 bis R8 unabhängig ein Wasserstoffatom, ein Halogenatom, eine Alkylgruppe, eine Alkoxygruppe, eine Alkylthiogruppe oder eine aromatische Gruppe sind, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können, wobei die Alkoxygruppe eine C₁-C₃₀-Alkoxygruppe ist, deren eines oder mehrere Kohlenstoffatome durch ein Sauerstoffatom, ein Stickstoffatom und/oder ein Schwefelatom substituiert sein können, und wobei die Alkylthiogruppe eine C₁-C₃₀-Alkylthiogruppe ist, deren eines oder mehrere Kohlenstoffatome durch ein Sauerstoffatom, ein Stickstoffatom und/oder ein Schwefelatom substituiert sein können; und
R`1 und R'2 eine Alkylgruppe sind, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können.

4. Verwendung nach Anspruch 1, wobei die Verbindung dargestellt wird durch die folgende allgemeine Formel (3): 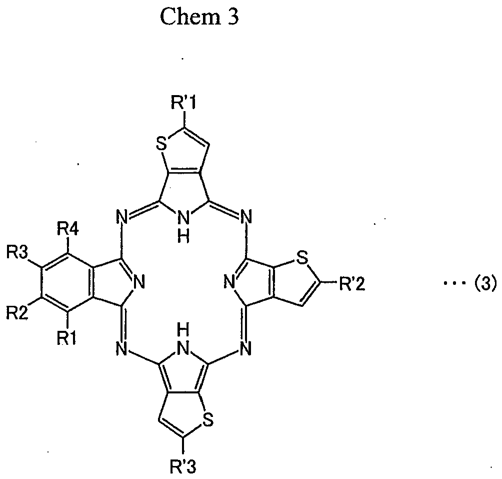 wobei R1 bis R4 unabhängig ein Wasserstoffatom, ein Halogenatom, eine Alkylgruppe, eine Alkoxygruppe, eine Alkylthiogruppe oder eine aromatische Gruppe sind, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können, wobei die Alkoxygruppe eine C₁-C₃₀-Alkoxygruppe ist, deren eines oder mehrere Kohlenstoffatome durch ein Sauerstoffatom, ein Stickstoffatom und/oder ein Schwefelatom substituiert sein können, und wobei die Alkylthiogruppe eine C₁-C₃₀-Alkylthiogruppe ist, deren eines oder mehrere Kohlenstoffatome durch ein Sauerstoffatom, ein Stickstoffatom und/oder ein Schwefelatom substituiert sein können; und
R'1 bis R'3 eine Alkylgruppe sind, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können.

5. Verwendung nach Anspruch 1, wobei die Verbindung durch die folgende allgemeine Formel (4) dargestellt wird: 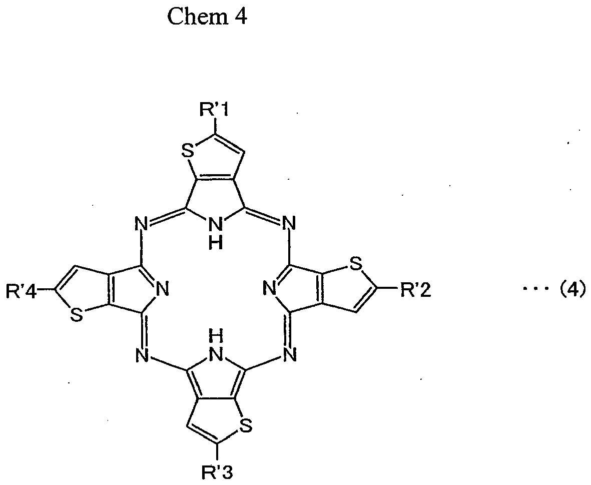 wobei R'1 bis R'4 eine Alkylgruppe sind, wobei die Alkylgruppe eine C₁-C₃₀-Alkylgruppe ist, deren eines oder mehrere Kohlenstoffatome durch ein Sauerstoffatom, ein Stickstoffatom und/oder ein Schwefelatom substituiert sein können.

6. Verwendung nach Anspruch 1, wobei die Verbindung dargestellt wird durch eine der folgenden chemischen Formel (5), (6) und (7): 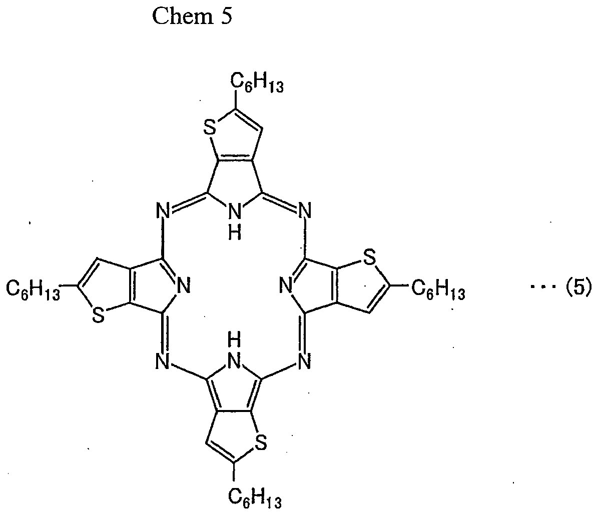 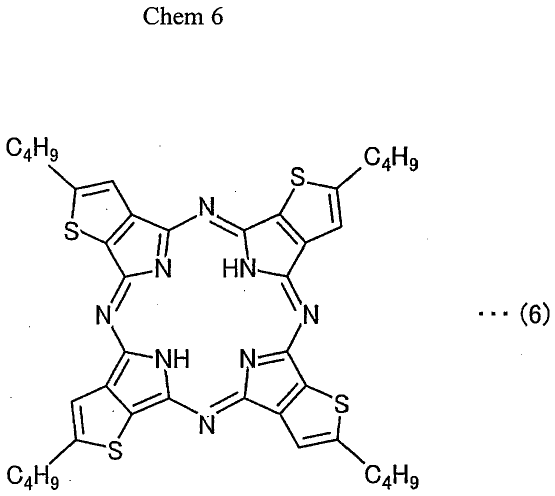 und

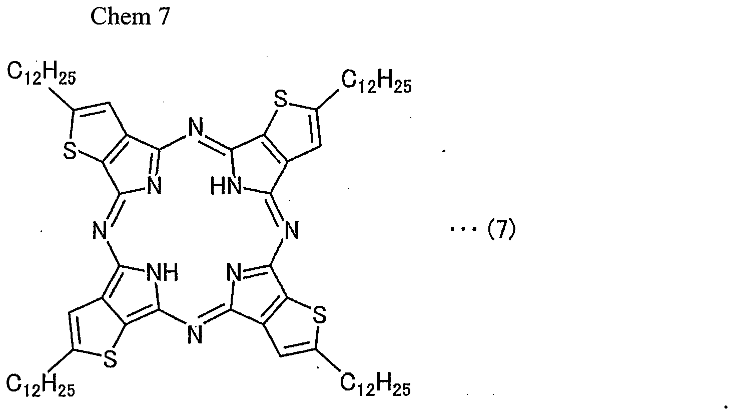

7. Verwendung nach einem der Ansprüche 1 bis 6, wobei das Porphyrazin der Verbindung ein Komplex mit einem Metallion bildet.

8. Vorrichtung, umfassend eine Verbindung nach einem der Ansprüche 1 bis 7, wobei die Vorrichtung ein photoelektrisches Umwandlungselement, eine Solarbatterie, ein Farbstoff-sensitiviertes Solarzellenelement, ein organisches EL-Element, ein Flüssigkristallanzeigeelement, ein organisches Dünnschicht-Transistor-Element oder eine Lichtemittierende Vorrichtung mit einer organischen Trägertransportschicht ist.

9. Vorrichtung nach Anspruch 8, wobei das organische Dünnschichttransistorelement ein Element vom Top-Kontakt-Typ ist.

10. Vorrichtung nach Anspruch 8, wobei das organische Dünnschichttransistorelement ein Element vom Bottom-Kontakt-Typ ist.

## Revendications

1. Utilisation d'un composé comme un matériau semi-conducteur organique, le composé étant un dérivé de porphyrazine formant un anneau condensé avec le thiophène ayant un squelette,
**caractérisé en ce que** le composé a sur le squelette du thiophène en position 2 un groupe alkyl étant un groupe alkyl en C₁-C₃₀ dont un ou plus des atomes de carbone peuvent être substitués avec un atome d'oxygène, un atome d'azote, et/ou un atome de soufre.

2. Utilisation telle que stipulée dans la revendication 1, le composé étant représenté par la formule générale suivante (1) : 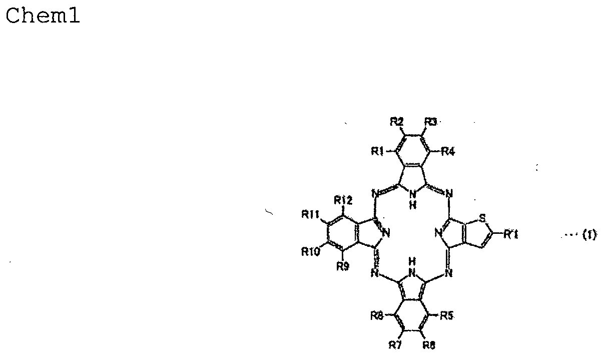 où :
R1 à R12 sont indépendamment un atome d'hydrogène, un atome d'halogène, un groupe alkyle, un groupe alkoxy, un groupe alkylthio, ou un groupe aromatique, le groupe alkyle étant un groupe alkyle en C₁-C₃₀ dont un ou plusieurs atomes de carbone peuvent être substitués avec un atome d'oxygène, un atome d'azote, et/ou un atome de soufre, le groupe alcoxy étant un groupe alcoxy en C₁-C₃₀ dont un ou plusieurs atomes de carbone peuvent être substitués avec un atome d'oxygène, un atome d'azote, et/ou un atome de soufre, et le groupe alkylthio étant un groupe alkythio en C₁-C₃₀ dont un ou plusieurs atomes de carbone peuvent être substitués avec un atome d'oxygène, un atome d'azote, et/ou un atome de soufre ; et
R'1 est un groupe alkyl, le groupe alkyl étant un groupe alkyl en C₁-C₃₀ dont un ou plusieurs atomes de carbone peuvent être substitués avec un atome d'oxygène, un atome d'azote, et/ou un atome de soufre.

3. Utilisation telle que stipulée dans la revendication 1, le composé étant représenté par la formule générale suivante (2) : 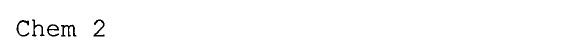 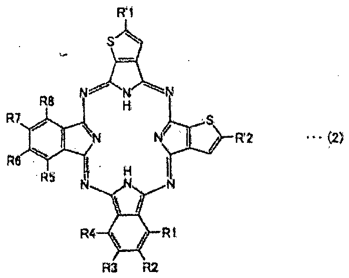 où :
R1 à R8 sont indépendamment un atome d'hydrogène, un atome d'halogène, un groupe alkyle, un groupe alcoxy, un groupe alkylthio, ou un groupe aromatique, le groupe alkyle étant un groupe alkyle en C₁-C₃₀ dont un ou plusieurs atomes de carbone peuvent être substitués avec un atome d'oxygène, un atome d'azote, et/ou un atome de soufre, le groupe alcoxy étant un groupe alcoxy en C₁-C₃₀ dont un ou plusieurs atomes de carbone peuvent être substitués avec un atome d'oxygène, un atome d'azote, et/ou un atome de soufre, et le groupe alkylthio étant un groupe alkythio en C₁-C₃₀ dont un ou plusieurs atomes de carbone peuvent être substitués avec un atome d'oxygène, un atome d'azote, et/ou un atome de soufre ; et
R'1 et R'2 sont un groupe alkyle, le groupe alkyle étant un groupe alkyle en C₁-C₃₀ dont un ou plusieurs atomes de carbone peuvent être substitués avec un atome d'oxygène, un atome d'azote et/ou un atome de soufre.

4. Utilisation telle que stipulée dans la revendication 1, le composé étant représenté par la formule générale suivante (3) : 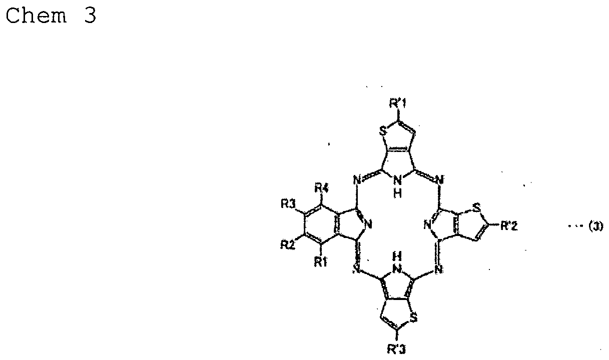 où R1 à R4 sont indépendamment un atome d'hydrogène, un atome d'halogène, un groupe alkyle, un groups alcoxy, un groupe alkylthio, ou un groupe aromatique, le groupe alkyle étant un groupe alkyle en C₁-C₃₀ dont un ou plusieurs atomes de carbone peuvent être substitués avec un atome d'oxygène, un atome d'azote, et/ou un atome de soufre, le groupe alcoxy étant un groupe alcoxy en C₁-C₃₀ dont un ou plusieurs atomes de carbone peuvent être substitués avec un atome d'oxygène, un atome d'azote, et/ou un atome de soufre, et le groupe alkylthio étant un groupe alkylthio en C₁-C₃₀ dont un ou plusieurs atomes de carbone peuvent être substitués avec un atome d'oxygène, un atome d'azote, et/ou un atome de soufre ; et
R'1 à R'3 sont un groupe alkyle, le groupe alkyle étant un groupe alkyle en C₁-C₃₀ dont un ou plusieurs atomes de carbone peuvent être substitués avec un atome d'oxygène, un atome d'azote et/ou un atome de soufre.

5. Utilisation telle que stipulée dans la revendication 1, le composé étant représenté par la formule générale suivante (4) ; 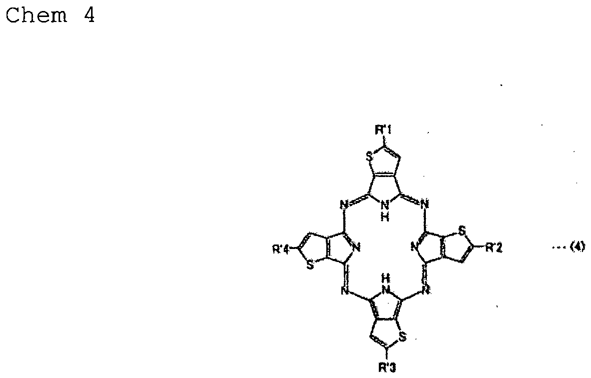 où R'1 à R'4 sont un groupe alkyle, le groupe alkyle étant un groupe alkyle en C₁-C₃₀ dont un ou plus d'atomes de carbone peuvent être substitués avec un atome d'oxygène, un atome d'azote, et/ou un atome de soufre.

6. Utilisation telle que stipulée dans la revendication 1, le composé étant représenté par l'une quelconque des formules chimiques suivantes (5), (6) et (7) : 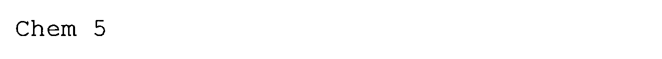 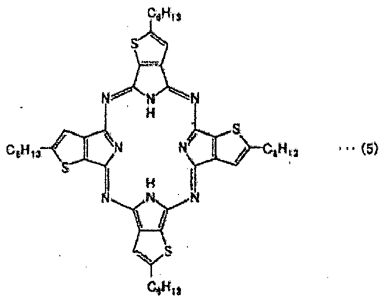 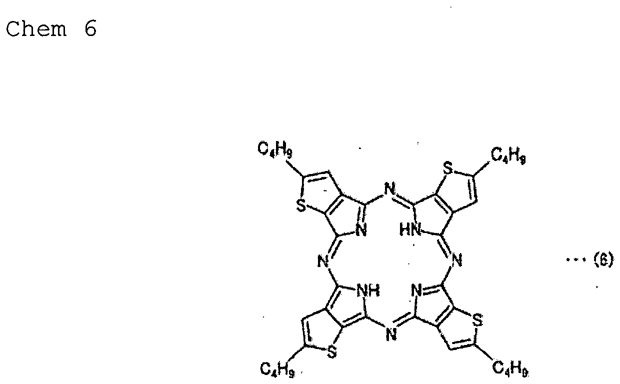 et

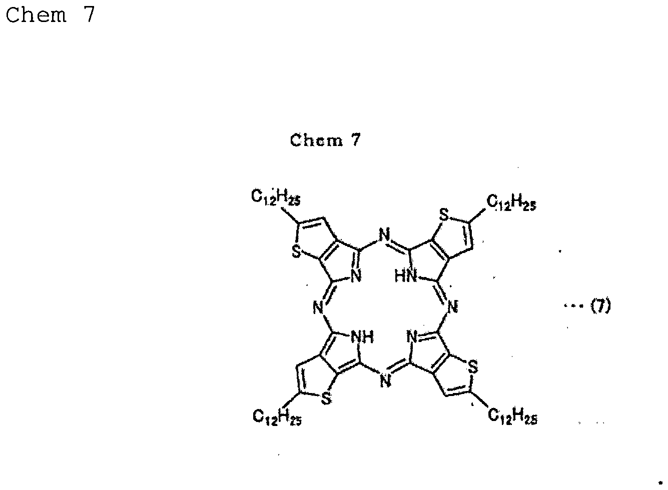

7. Utilisation telle que stipulée dans l'une quelconque des revendications 1 à 6, dans lequel la porphyrazine du composé forme un complexe avec un ion métallique.

8. Dispositif comprenant un composé tel que stipulé dans l'une quelconque des revendications 1 à 7 ; le dispositif étant un élément de conversion photoélectrique, une batterie solaire, un élément de cellule solaire sensibilisée par un colorant, un élément électroluminescent organique, un élément d'affichage à cristaux liquides, un élément transistor à couche mince organique, ou un dispositif d'émission de lumière ayant une couche de transport de support organique.

9. Dispositif tel que stipulé dans la revendication 8, dans lequel l'élément transistor à couche mince organique est un élément de type contact supérieur.

10. Dispositif tel que stipulé dans la revendication 8, dans lequel l'élément transistor à couche mince organique est un élément de type contact inférieur.
